(19) **Europäisches Patentamt / European Patent Office / Office européen des brevets**

(11) **EP 4 314 854 B1**

(12) **FASCICULE DE BREVET EUROPEEN**

(45) Date de publication et mention de la délivrance du brevet:
**02.07.2025 Bulletin 2025/27**

(21) Numéro de dépôt: **22747362.6**

(22) Date de dépôt: **28.06.2022**

(51) Classification Internationale des Brevets (IPC):
**G01R 33/56** *(2006.01)*   **G01R 33/563** *(2006.01)*

(52) Classification Coopérative des Brevets (CPC):
**G01R 33/5608;** G01R 33/56316; G01R 33/56341; G01R 33/5635; G01R 33/56366

(86) Numéro de dépôt international:
**PCT/FR2022/051278**

(87) Numéro de publication internationale:
**WO 2023/275478 (05.01.2023 Gazette 2023/01)**

(54) **PROCÉDÉ D'ATTÉNUATION DU BRUIT D'IMAGES RÉSULTANT D'ACQUISITIONS MULTIPLES PAR RÉSONANCE MAGNÉTIQUE**

VERFAHREN ZUR RAUSCHUNTERDRÜCKUNG IN BILDERN AUS MEHREREN MRT-ERFASSUNGEN

METHOD FOR ATTENUATING NOISE IN IMAGES RESULTING FROM MULTIPLE MRI ACQUISITIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.06.2021 FR 2107003**

(43) Date de publication de la demande:
**07.02.2024 Bulletin 2024/06**

(73) Titulaire: **Olea Medical**
**13600 La Ciotat (FR)**

(72) Inventeurs:
• **CASAGRANDA, Stefano**
**20060 BUSSERO (IT)**
• **PAPAGEORGAKIS, Christos**
**13600 LA CIOTAT (FR)**

(74) Mandataire: **Brun, Philippe Alexandre Georges**
**Med'inVent Consulting**
**Espace Mistral - Bât.A**
**297 avenue du Mistral**
**ZI ATHELIA IV**
**13705 La Ciotat Cedex (FR)**

(56) Documents cités:
• **VERAART JELLE ET AL: "Denoising of diffusion MRI using random matrix theory", NEUROIMAGE, vol. 142, 11 August 2016 (2016-08-11), AMSTERDAM, NL, pages 394 - 406, XP055897412, ISSN: 1053-8119, DOI: 10.1016/ j.neuroimage.2016.08.016**
• **BREITLING JOHANNES ET AL: "Adaptive denoising for chemical exchange saturation transfer MR imaging", NMR IN BIOMEDICINE., vol. 32, no. 11, 30 July 2019 (2019-07-30), GB, XP055897513, ISSN: 0952-3480, DOI: 10.1002/ nbm.4133**
• **ZHANG YONGQIN ET AL: "Joint image denoising using adaptive principal component analysis and self-similarity", INFORMATION SCIENCES, ELSEVIER, AMSTERDAM, NL, vol. 259, 11 August 2013 (2013-08-11), pages 128 - 141, XP028791534, ISSN: 0020-0255, DOI: 10.1016/ J.INS.2013.08.002**
• **MANJÓN JOSÉ V. ET AL: "Diffusion Weighted Image Denoising Using Overcomplete Local PCA", PLOS ONE, vol. 8, no. 9, 3 September 2013 (2013-09-03), pages e73021, XP055898567, DOI: 10.1371/journal.pone.0073021**

## Description

L'invention concerne un procédé d'atténuation du bruit (ou de « débruitage ») d'images obtenues par acquisitions multiples via un appareil d'imagerie par résonance magnétique.

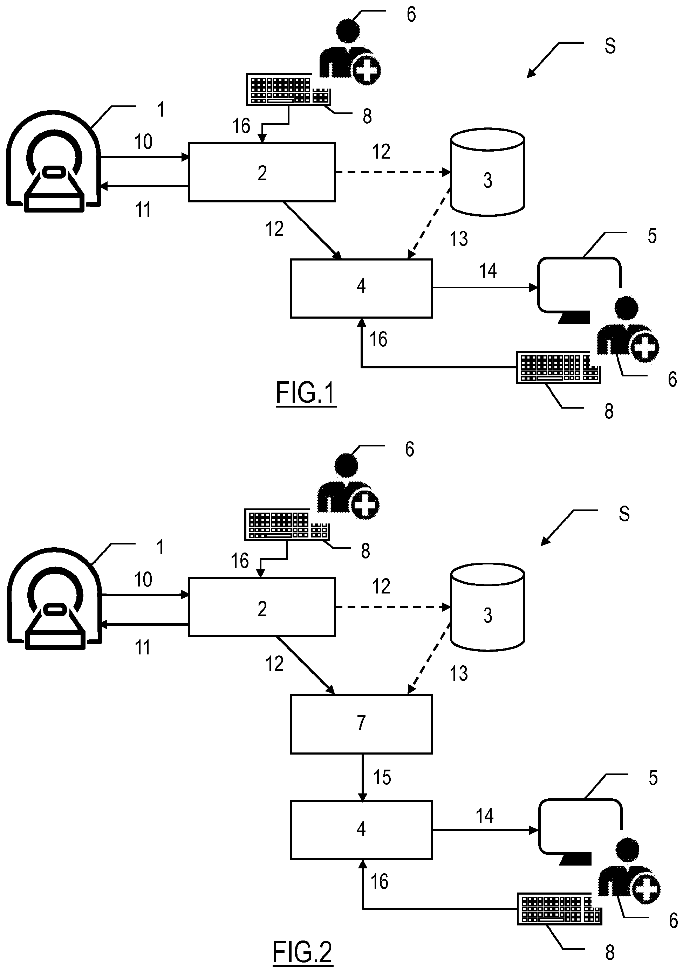

L'imagerie par résonance magnétique, également connue sous l'acronyme français IRM ou anglo-saxon MRI (pour « Magnetic Resonance Imaging »), est basée sur une analyse de la réponse d'un proton d'une molécule d'eau lorsqu'il est excité dans un champ magnétique. Cette réponse dépend de l'environnement d'un tel proton et permet de différencier plusieurs types de tissus. Un appareil d'imagerie 1 par résonance magnétique nucléaire d'un système d'analyse d'imagerie S, tel qu'illustré à titre d'exemple non limitatif par les figures 1 et 2, est généralement utilisé. Celui-ci délivre une pluralité de séquences d'images numériques 12 d'une ou plusieurs parties du corps d'un patient, à titre d'exemples non limitatifs, le cerveau, le cœur, les poumons. Ledit appareil 1 applique pour cela une combinaison d'ondes électromagnétiques à hautes fréquences sur la partie du corps considérée et mesure le signal réémis par certains atomes, tels qu'à titre d'exemple non limitatif, l'hydrogène pour l'imagerie par résonance magnétique nucléaire. L'appareil permet ainsi de déterminer les propriétés magnétiques et, par voie de conséquence, la composition chimique des tissus biologiques et donc leurs natures, en chaque volume élémentaire, que l'on nomme communément un voxel, du volume imagé. L'appareil d'imagerie par résonance magnétique 1 est commandé à l'aide d'une console 2. Un utilisateur 6, par exemple un opérateur, praticien ou chercheur, peut ainsi choisir des commandes 11 pour piloter l'appareil 1, à partir de paramètres ou de consignes 16 saisis via une interface homme-machine d'entrée 8 du système d'analyse. Une telle interface homme-machine 8 peut consister par exemple en un clavier informatique, un dispositif de pointage, d'un écran tactile, un microphone ou, plus généralement, toute interface agencée pour traduire une gestuelle ou une consigne émise par un humain 6 en données de commande ou de paramétrage. A partir d'informations 10 produites par ledit appareil 1, on obtient une pluralité de séquences d'images numériques 12 d'une partie d'un corps humain ou animal. Nous nommerons également « données expérimentales » de telles informations 10 ou images 12.

Les séquences d'images 12 peuvent optionnellement être stockées au sein d'un serveur 3, c'est-à-dire un ordinateur doté de moyens de mémorisation propres et constituer un dossier médical 13 d'un patient. Un tel dossier 13 peut comprendre des images de différents types, telles que des images fonctionnelles mettant en évidence l'activité des tissus ou des images anatomiques reflétant les propriétés des tissus. Les séquences d'images 12 ou, plus généralement les données expérimentales, sont analysées par une unité de traitement 4 agencée à cette fin. Une telle unité de traitement 4 peut, par exemple, consister en un ou plusieurs microprocesseurs ou microcontrôleurs mettant en œuvre des instructions de programmes d'application idoines chargées dans des moyens de mémorisation du système d'analyse d'imagerie S.

On entend par « moyens de mémorisation » toute mémoire informatique volatile ou, avantageusement, non volatile. Une mémoire non volatile est une mémoire informatique dont la technologie permet de conserver ses données en l'absence d'une alimentation en énergie électrique. Elle peut contenir des données résultant de saisies, de calculs, de mesures et/ou des instructions de programmes. Les principales mémoires non volatiles actuellement disponibles sont de type inscriptible électriquement, telles que l'EPROM (« Erasable Programmable Read-Only Memory », selon une terminologie anglo-saxonne) ou encore inscriptibles et effaçables électriquement, telle que l'EEPROM (« Electrically-Erasable Programmable Read-Only Memory), flash, SSD (« Solid-State Drive », selon une terminologie anglo-saxonne), etc. Les mémoires non volatiles se distinguent des mémoires dites « volatiles » dont les données sont perdues en l'absence d'une alimentation électrique. Les principales mémoires volatiles actuellement disponibles sont de type RAM (« Random Access Memory » selon une terminologie anglo-saxonne ou encore nommée « mémoire vive »), DRAM (mémoire vive dynamique, nécessitant une réactualisation régulière), SRAM (mémoire vive statique nécessitant une telle réactualisation lors d'une sous-alimentation électrique), DPRAM ou VRAM (particulièrement adaptées à la vidéo), etc. Une « mémoire de données », dans la suite du document, peut être volatile ou non volatile selon l'application visée.

L'unité de traitement 4 comporte des moyens de communication avec le monde extérieur afin de recueillir les images. Lesdits moyens de communication permettent en outre à l'unité de traitement 4 de délivrer, *in fine,* un rendu, par exemple graphique et/ou sonore, d'une estimation ou d'une quantification d'un biomarqueur élaborée par ladite unité de traitement 4 à partir des données expérimentales 10 et/ou 12 obtenues par imagerie par résonance magnétique, à un utilisateur 6 du système d'analyse d'imagerie S par l'intermédiaire d'une interface homme-machine de sortie 5. Dans tout le document, on entend par « interface homme-machine de sortie », tout dispositif, employé seul ou en combinaison, permettant de sortir ou délivrer une représentation graphique, haptique, sonore ou, plus généralement, perceptible par l'humain, d'un signal physiologique reconstruit, en l'espèce un biomarqueur, à un utilisateur 6 d'un système d'analyse d'Imagerie par résonance magnétique S. Une telle interface homme-machine de sortie 5 peut consister de manière non exhaustive en un ou plusieurs écrans, haut-parleurs ou autres moyens alternatifs adaptés. Ledit utilisateur 6 du système d'analyse d'imagerie S peut ainsi confirmer ou infirmer un diagnostic, décider d'une action thérapeutique qu'il jugera adéquate, approfondir des travaux de recherche, peaufiner des paramètres de réglage d'un équipement de mesure, etc. Optionnellement, cet utilisateur 6 peut également paramétrer le fonctionnement de l'unité de traitement 4 ou de l'interface homme-machine de sortie 5 au moyen de paramètres 16 de fonctionnement et/ou d'acquisition. Par exemple, il peut ainsi définir des seuils d'affichage ou choisir les biomarqueurs, indicateurs ou paramètres estimés ou quantifiés pour lesquels il souhaite disposer d'une représentation. L'utilisateur exploite pour cela l'interface homme-machine d'entrée 8 précédemment évoquée ou une deuxième interface d'entrée prévue pour cela. Avantageusement, les interfaces homme-machine d'entrée 8 et de sortie 5 peuvent ne constituer qu'une seule et même entité physique. Lesdites interfaces homme-machine d'entrée 8 et de sortie 5 du système d'analyse d'imagerie peuvent également être intégrées à la console d'acquisition 2. Il existe une variante, décrite en liaison avec la figure 2, pour laquelle un système d'imagerie, tel que décrit précédemment, comporte en outre une unité de prétraitement 7 pour analyser les séquences d'images 12, en déduire des signaux expérimentaux 15 et délivrer ces derniers à l'unité de traitement 4 qui est ainsi déchargée de cette tâche.

Plusieurs images en deux dimensions ou volumes en trois dimensions peuvent être acquis avec ou sans modification des réglages et des paramètres du système d'imagerie médicale S. Par exemple, pour étudier la réaction temporelle à l'arrivée d'un agent de contraste, on peut simplement répéter l'acquisition sans modifier les réglages et les paramètres dudit système. En revanche, d'autres techniques d'imagerie nécessitent de modifier lesdits réglages et/ou paramètres pour échantillonner l'espace de paramètres souhaité.

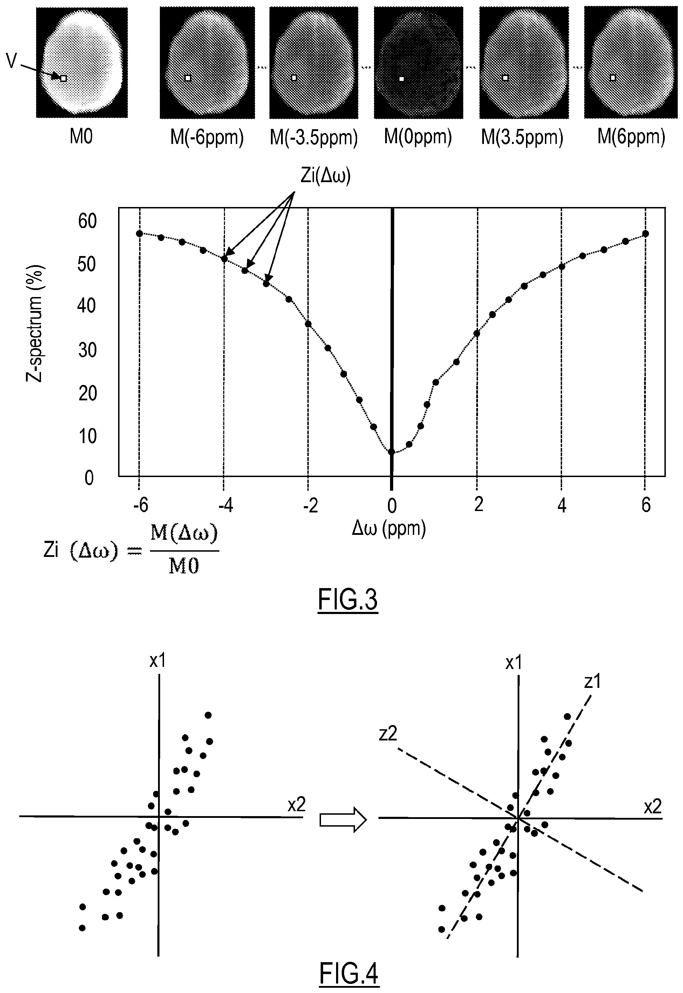

Parmi les techniques ou modalités basées sur l'imagerie par résonance magnétique, on distingue l'imagerie par transfert de saturation d'échanges chimiques ou CEST pour *Chemical Exchange Saturation Transfer,* selon une terminologie anglo-saxonne. Une telle technique consiste à appliquer une impulsion radiofréquence selon différentes fréquences de résonance déterminées $\omega$, de sorte que des espèces chimiques d'intérêt atteignent un état de saturation. Lesdites fréquences de résonance déterminées $\omega$ sont en effet respectivement associées à différentes espèces chimiques d'intérêt, telles que, de manière non limitative, le groupe amide (-NH), le groupe amine (-NH2) ou le groupe hydroxyle (-OH). Leurs protons d'hydrogènes labiles ainsi excités sont échangés avec des protons d'hydrogène non excités de l'eau. Une telle application d'une impulsion radiofréquence, à une fréquence de résonance déterminées $\omega$ autre que celle $\omega 0$ associée à l'eau, continuellement répétée pendant une durée déterminée, par exemple quelques secondes, conduit à une accumulation de saturation en eau d'une espèce chimique d'intérêt. Une concentration de cette dernière peut être indirectement mesurée par la diminution du signal de l'eau, phénomène appelé l' « effet CEST » qui est analysé sous la forme d'un spectre Z (« Z-spectrum » selon une terminologie anglo-saxonne). Une telle diminution peut être facilement détectée par des séquences d'acquisition d'imagerie par résonance magnétique rapides telles que, de manière non exhaustive, l'imagerie écho-planaire à coup unique également connue sous la terminologie anglo-saxonne « single shot echo-planar imaging ». La figure 3 présente, pour un voxel V d'intérêt, un exemple d'un spectre Z sous la forme d'un ensemble d'échantillons $Zi(\Delta\omega)$ (ou « data set » selon une terminologie anglo-saxonne) selon des décalages fréquentiels relatifs $\Delta\omega$ par rapport à la fréquence de l'eau, de tels décalages $\Delta\omega$ étant exprimés en ppm. Plus précisément, ladite figure 3 illustre différentes images M(-6ppm), M(-3.5ppm), M(0ppm), M(3.5ppm), M(6ppm) correspondant à des décalages fréquentiels $\Delta\omega$ respectivement égaux à -6 ppm, 3.5 ppm, 0 ppm, 3.5 ppm et 6 ppm. Elle présente en outre une image M0 acquise pour des fréquences éloignées de la fréquence de l'eau. Le spectre $Z(\Delta\omega)$, que l'on peut qualifier de « normalisé », correspond, pour un voxel donné, au ratio $M(\Delta\omega)$ sur M0, pour des décalages fréquentiels compris entre -6 ppm et 6ppm. Lorsque les échantillons $Zi(\Delta\omega)$ sont ordonnés selon des $\Delta\omega$ croissants, comme l'indique la figure 3, ledit spectre Z peut être décrit sous la forme d'un signal discret décrivant des m mesures, avantageusement normalisées par un signal mesuré sans saturation par radiofréquence et exprimées en pourcentages, de la magnitude d'un signal expérimental délivré par un appareil de mesure 1 pour un volume élémentaire d'un organe. Selon la figure 3, cet ensemble de m échantillons, lorsque ces derniers sont ordonnés selon des décalages fréquentiels $\Delta\omega$ croissants, constitue un signal discret Z décrivant sensiblement un 'V' dont le minimum $Zi(\Delta\omega)m$ est associé au décalage fréquentiel $\Delta\omega$ =0 ppm, lorsque l'appareil est parfaitement réglé et/ou lorsque le champ magnétique statique était homogène.

La technique CEST améliore la détection de certains métabolites du corps humain dont la concentration est insuffisante pour être détectée par des séquences d'imagerie par résonance magnétique traditionnelles. Cette technique CEST permet ainsi d'obtenir des informations précieuses pour un praticien cherchant à établir un diagnostic et à prendre une décision thérapeutique dans le traitement de pathologies.

Nous pouvons également citer l'imagerie par tenseur de diffusion, également connue sous l'acronyme anglo-saxon DTI pour « Diffusion Tensor Imaging » pour laquelle des données sont acquises pour différentes intensités (valeurs b) et directions du gradient de diffusion magnétique. Dans cette catégorie se trouve l'imagerie de perfusion, également connue sous l'acronyme anglo-saxon PWI pour « Perfusion Weighted Imaging » et deux de ses principales techniques : le contraste dynamique de susceptibilité, connu sous l'acronyme anglo-saxon DSC pour « Dynamic Susceptibility Contrast » et le contraste dynamique amélioré, connu sous l'acronyme anglo-saxon DCE pour « Dynamic Contrast Enhanced ».

Il existe également la technique d'imagerie de diffusion à haute résolution angulaire également connue sous l'acronyme anglo-saxon HARDI pour « High Angular Resolution Diffusion Imaging ».

En imagerie par résonance magnétique, le signal induit dans une bobine réceptrice est un signal complexe continu, c'est-à-dire qu'il comporte une partie réelle et une partie imaginaire, acquis dans un domaine de fréquences communément appelé « espace k » ou « k-space » selon une terminologie anglo-saxonne. Le bruit sur un tel signal peut être décrit comme une contribution complexe, additive et non corrélée audit signal pur. Un tel bruit comporte donc une composante réelle et une composante imaginaire indépendantes l'une de l'autre et distribuées de manière identique suivant une distribution gaussienne avec une moyenne nulle et un écart-type (cf. Cárdenas-Blanco et al. 2008 ; Aja-Fernández et Vegas-Sánchez-Ferrero 2016 - enseignement technique accessible par exemple via l'hyperlien « https://doi.org/10.1007/978-3-319-39934-8 »). Cependant, il est courant dans ce domaine de considérer des images de magnitude plutôt que ledit signal complexe. Étant donné que la transformation du signal complexe en signal de magnitude enregistré dans les images de magnitude est non linéaire, la distribution des intensités des pixels dans les images résultantes n'est généralement pas gaussienne et, dans certaines conditions, elle peut être modélisée par une distribution de Rice, comme le précise le document Cárdenas-Blanco, Arturo, Cristian Tejos, Pablo Irrarrazaval, and Ian Cameron - 2008, « Noise in Magnitude Magnetic Resonance Images. » Concepts in Magnetic Resonance Part A 32A (6) : 409-16, document consultable via le lien « https://doi.org/10.1002/cmr.a.20124 ».

Dans le domaine de l'imagerie par résonance magnétique, il est connu de réduire ou de supprimer le bruit des données expérimentales (c'est-à-dire des données mesurées). On utilise les termes « débruiter » ou « débruitage » pour décrire cette opération qui consiste à rejeter ou filtrer le bruit pour ne considérer que les informations importantes au sein desdites données expérimentales. Pour cela, de nombreuses méthodes exploitant l'analyse en composantes principales également connue sous l'acronyme ACP, voire l'acronyme anglo-saxon PCA pour « Principal Component Analysis ».

L'analyse en composantes principales est une technique non paramétrique (c'est-à-dire ne faisant appel à aucun modèle) conçue à l'origine pour réduire les dimensions d'un ensemble de données, tout en conservant l'essence desdites données. Une telle approche est divulguée par exemple dans le document Jolliffe, I. 2002. « Principal Component Analysis ». Second. Springer-Verlag, accessible par le lien « https://doi.org/10.1007/b98835 ». Une telle méthode identifie et exploite les corrélations linéaires dans les données considérées et en extrait un nouvel ensemble de données non corrélées, de taille plus restreinte, appelé « composantes principales ». Ces composantes principales ainsi extraites sont ordonnées en fonction de la variabilité (ou variances) qu'elles expriment. Ainsi, les premières composantes principales décrivent la partie la plus informative des données, concentrant la majeure partie de la variance des données.

En termes simples, appliquée à l'imagerie, le but de l'analyse en composantes principales est d'identifier la base la plus significative pour « réexprimer » des données expérimentales bruitées et constituer un ensemble de données expérimentales débruitées, c'est-à-dire pour lequel le bruit a été filtré afin de révéler une ou plusieurs structures d'intérêt éventuellement masquées par ledit bruit.

D'un point de vue géométrique, le problème de l'ACP peut être simplifié comme suit. Pour une collection donnée de points (réels) dans un espace à m dimensions, trouver q vecteurs pour lesquels :

- un $j^{ème}$ vecteur représente une droite qui s'ajuste le mieux aux points ;
- ledit $j^{ème}$ vecteur étant orthogonal aux j-1 vecteurs calculés précédemment.

On entend par « meilleur ajustement » le fait de trouver une ligne ou axe qui minimise la distance quadratique moyenne des points à la ligne. Un tel ajustement est illustré par la figure 4. Selon cette figure, nous pouvons constater, dans la partie de gauche, un nuage de points s'inscrivant dans un repère ou une base en deux dimensions illustré(e) par les axes x1 et x2. Nous pouvons constater que, sur la partie droite, l'axe z1 est la ligne qui s'ajuste le mieux auxdits points, les données ou points pouvant être projetés dans un nouveau repère ou base décrit(e) par l'axe z1 et l'axe z2 normal à z1. La composante principale z1 est caractérisée par la plus grande variance λ1 que l'on nomme également « valeur propre » ou « eigenvalue » selon une terminologie anglo-saxonne. Ainsi, l'ensemble Λ des valeurs propres respectives λ1 et λ2 des composantes principales z1 et z2 représente les quantités d'informations recueillies par lesdites composantes principales ou leurs capacités informatives respectives. On nomme « vecteurs propres » Φ={φ1, φ2} ou « eigenvectors » selon une terminologie anglo-saxonne, les composantes principales z1 et z2 sur la figure 4.

Ainsi, une analyse en composantes principales sous-entend que :

- les données expérimentales de dimension m peuvent être exprimées sous forme de combinaisons linéaires de vecteurs;
- la variance λ de chaque composante principale indique des informations significatives ;
- les q composantes principales, formant la nouvelle base ou le nouveau repère, sont orthogonales.

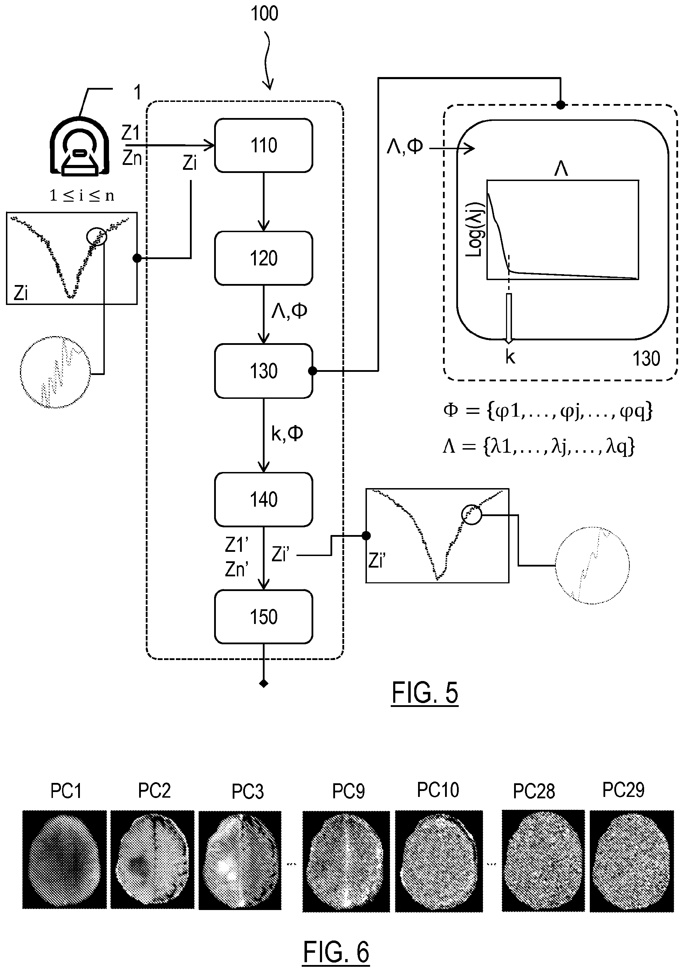

La figure 5 illustre un procédé connu pour atténuer le bruit ou « débruiter » des données expérimentales sous la forme de spectres Z normalisés (de m phases ou fréquences chacun) pour une collection de n emplacements spatiaux (encore appelés « voxels ») d'un cerveau humain. Ainsi, pour un voxel donné, les données expérimentales sont un vecteur de m acquisitions ou échantillons. Un tel procédé 100 comporte :

- une première étape 110 d'acquisition ou de collecte de données expérimentales Z1 à Zn pour un ensemble de n voxels V1 à Vn à m phases différentes (ou échantillons) desdites données de voxels sous la forme d'une matrice de Casorati C (matrice de n lignes sur m colonnes, pour laquelle chacune des n lignes contient m valeurs acquises pour un voxel donné. Les n lignes sont dédiées respectivement aux différents voxels d'un segment ou volume considéré ;
- une étape 120 pour extraire ou calculer les q = m composantes principales décrivant au mieux cet ensemble de données expérimentales bruitées, en l'espèce pour un voxel donné, le spectre Zi tel que :

$$Zi = Za + \sum_{j=1}^{q} (Zi - Za)\, \varphi_j^T \varphi_j$$

Za étant la moyenne du spectre Zi; pour cela, est calculé :

$$\mathrm{cov}(C) = \Phi^T \Lambda \Phi$$

Φ décrivant les vecteurs propres (ou « eigenvectors » selon une terminologie anglo-saxonne) des composantes principales et Λ leurs valeurs propres (ou « eigenvalues » selon une terminologie anglo-saxonne) ; les q composantes, donc leurs vecteurs propres φ1 à φq, extraites ou calculées, sont classées ou ordonnées généralement selon leurs variances ou valeurs propres respectives. La variance d'une composante principale de vecteur propre φj est décrite par une valeur scalaire λj, que l'on nomme « valeur propre » ;

- une étape 130 de détermination du nombre optimal k<q de composantes principales ou, plus précisément, de sélection des k composantes les plus significatives ou informatives parmi les q composantes extraites, c'est-à-dire les k composantes décrivant des informations spatiales d'intérêt par opposition aux q-k autres composantes décrivant principalement du bruit et qui méritent d'être écartées ;
- une étape 140 de projection des données expérimentales bruitées sur les k composantes restantes pour constituer un nouvel ensemble de données expérimentales débruitées, en l'espèce sur l'exemple de la figure 5, un spectre Zi' pour un voxel d'intérêt, tel que :

$$Zi' = Za + \sum_{j=1}^{k} (Zi - Za)\, \varphi_j^T \varphi_j$$

- une étape 150 de réorganisation des données expérimentales débruitées Z1' à Zn' dans l'espace d'origine des voxels V1 à Vn et d'exploitation de tout ou partie desdites données expérimentales débruitées Z1', ..., Zi', ..., à Zn' selon l'application choisie.

A titre d'exemple, comme l'indique la figure 5, le spectre bruité Zi d'un voxel considéré apparaît, après la mise en œuvre d'un tel procédé 100, sous la forme d'une courbe Zi' plus « douce » que celle décrite par les données expérimentales « brutes » Zi.

L'étape 120 a été décrite selon un premier mode de réalisation à partir de la matrice de covariance. En variante, une telle production de q composantes principales peut consister selon un deuxième mode de réalisation, en la décomposition en valeurs singulières (« Singular Value Decomposition » ou SVD selon une terminologie anglo-saxonne) d'une matrice de Casorati C telle qu'évoquée précédemment. Selon ce deuxième mode de réalisation, une donnée expérimentale, par exemple sous la forme d'un spectre Zi, pour un voxel donné peut s'exprimer sous la forme :

$$Zi = Za + \sum_{j=1}^{q} t_{ij} v_j = Za + t_i V^t$$

où Za est le spectre moyen, c'est-à-dire la moyenne des valeurs de la ième ligne de la matrice de Casorati C, $t_i$ est un vecteur de q coefficients, ci-après nommés « scores » pour décrire les contributions respectives des composantes principales et $V^t$ est une matrice de q lignes et q colonnes desdites composantes principales. Ainsi, nous pouvons réécrire la matrice C telle que $C = TV^t + Za$ qui est calculée à partir d'une décomposition en valeurs singulières de la matrice C telle que $C - Za = USV^t$ où U est une matrice de n lignes et n colonnes décrivant les scores (ou coefficients ou encore poids) des composantes principales $u_j$ décrit les scores de composante principale de rang j), S est une matrice de n lignes et q colonnes décrivant q valeurs $S_1$ à $S_q$ non nulles appelées « valeurs singulières » and $V^t$ est une matrice de m lignes et m colonnes décrivant la nouvelle base des composantes principales.

Les valeurs singulières de la matrice S sont classées selon un ordre décroissant. Notons que si la matrice C est transposée, ses lignes décrivant m variables et ses colonnes n échantillons, alors les interprétations respectives des matrices U et V sont interchangées.

En identifiant les termes dans les précédentes équations, on peut déduire que T = US est une matrice des scores des composantes principales multipliés par des valeurs singulières, $V^t$ étant la matrice des composantes principales. Un tel deuxième mode de réalisation de l'étape 120 est principalement axé sur l'analyse de l'information spatiale contenue dans chaque colonne de la matrice U ou, de manière similaire, de la matrice T. Comme évoqué précédemment, une telle étape 120 peut être basée sur la matrice de covariance $\text{cov}(C) = \Phi^T \Lambda \Phi$ pour laquelle $\Phi$ décrit les vecteurs propres des composantes principales et $\Lambda$ leurs valeurs propres ; les q composantes, donc leurs vecteurs propres $\varphi 1$ à $\varphi q$, extraites ou calculées, sont classées ou ordonnées généralement selon leurs variances ou valeurs propres respectives $\lambda j$. Il existe une relation directe entre les valeurs singulières selon le deuxième mode de réalisation et les valeurs propres selon le premier mode de réalisation obtenues (c'est-à-dire à partir de la matrice de covariance) telle que

$$\lambda j = \frac{s_j^2}{n-1}.$$

Pour atténuer le bruit de données expérimentales, en l'espèce un spectre bruité Zi, ladite donnée expérimentale « débruitée » s'obtient par la mise en œuvre de l'étape 140 de projection des données expérimentales bruitées sur les k composantes restantes pour constituer un nouvel ensemble de données expérimentales débruitées, en l'espèce sur l'exemple de la figure 5, un spectre Zi' pour un voxel d'intérêt, tel que :

$$Zi' = Za + \sum_{j=1}^{k} t_{ij} v_j = Za + t_i' V^{t'}$$

pour laquelle ti' est un vecteur de k scores et $V^{t'}$ est une matrice de k lignes et q colonnes des composantes principales.

Parmi les étapes du débruitage par analyse des composantes principales, l'étape 130 de sélection des composantes est la plus délicate. Pour obtenir les informations spatiales cachées dans les composantes principales, on peut remodeler chaque composante principale (c'est-à-dire les valeurs réelles des voxels projetées sur un vecteur propre) dans sa dimension d'origine (c'est-à-dire dans un volume ou une tranche), comme le montre la figure 6. Les images PC1 à PC29 associées respectivement auxdites composantes principales de rangs 1 à 29 décrivent les scores associés aux vecteurs propres $\varphi 1$ à $\varphi 29$ pour l'ensemble des voxels V1 à Vn considérés selon le mode de production 120 des composantes principales retenu. De tels scores correspondent à la projection des données expérimentales sur lesdites vingt-neuf composantes principales. Par mesures de simplification, nous utiliserons le terme « score » pour décrire une contribution d'une composante principale, quel que soit le mode de production ou d'extraction 120 d'une telle composante principale. Le principe d'une analyse en composantes principales, selon l'état de l'art, revient en quelque sorte à chercher à exprimer au mieux les signaux ou données expérimentaux d'un ensemble de voxels découlant d'une acquisition multiple, sous la forme de combinaisons linéaires de composantes principales selon des scores respectifs, lesdites composantes principales étant classées selon un ordre de prépondérance découlant directement de valeurs propres desdites composantes principales ou de valeurs singulières qui leur sont associées. Ainsi, l'image PC1 de la figure 6 décrit, à l'instar d'une image d'une tranche d'un cerveau humain, les scores de la première composante principale, pour reconstituer les courbes expérimentales (spectres Zi pour i compris entre 1 et n) pour n voxels considérés. De la même manière l'image PC2 de la figure 6 décrit, à l'instar d'une image d'une tranche d'un cerveau humain, les scores de la deuxième composante principale pour reconstituer les courbes expérimentales des voxels considérés. En d'autres termes, l'image PC2 de la figure 6 décrit lesdites données expérimentales Z1 à Zn projetées sur la deuxième composante principale. Il en est de même pour les images PC3 à PC29 décrivant respectivement les scores de la troisième jusqu'à la vingt-neuvième composante principale pour les voxels considérés. Cette figure 6 permet d'illustrer le résultat de la mise en œuvre de l'étape 120 précédemment évoquée. Comme nous pouvons le constater à l'œil nu, les neuf premières images PC1 à PC9 respectivement associées aux composantes principales de rangs 1 à 9 semblent, selon un degré décroissant, exprimer une information spatiale pertinente par opposition aux autres images PC10 à PC29 respectivement associées aux composantes principales de rangs supérieurs qui semblent ne plus rien indiquer du tout. Bien évidemment, la sélection 130 de k composantes principales significatives ne se fait pas à l'œil nu mais selon un procédé automatique.

L'état de l'art regorge de procédés ou de méthodes utilisant différents modèles et hypothèses mathématiques ou statistiques, des combinaisons avec d'autres techniques et divers critères pour déterminer un sous-ensemble « optimal » de k composantes dans de nombreux domaines et applications. Parmi celles-ci, certaines ont été utilisées avec succès à des fins de débruitage ou de filtrage en imagerie par résonance magnétique, en reconstruisant généralement l'ensemble de données expérimentales à l'aide des composantes informatives et en rejetant les composantes liées principalement au bruit. A cette fin, de nombreuses approches axées sur les données ont été proposées pour déterminer les k composantes principales parmi q composantes principales extraites qu'il est pertinent de préserver et q-k composantes principales à exclure lors la reconstruction des données expérimentales pour obtenir des données expérimentales débruitées.

Cependant, la plupart des critères de sélection sont basés sur l'ensemble $\Lambda$ des valeurs propres associées aux q composantes principales extraites, c'est-à-dire basés sur des mesures de variances exprimées par l'ensemble des données originales, composante par composante, ou sur les résidus, c'est-à-dire sur les différences mathématiques respectives entre les données originales et les données reconstruites. Nous pouvons citer à cet égard, et de manière non exhaustive, le document Breitling, Johannes, Anagha Deshmane, Steffen Goerke, Andreas Korzowski, Kai Herz, Mark E. Ladd, Klaus Scheffler, Peter Bachert, and Moritz Zaiss - 2019 - « Adaptive Denoising for Chemical Exchange Saturation Transfer MR Imaging. » NMR in Biomedicine, no. March 2019: 1-14, accessible par le lien « https://doi.org/ 10.1002/nbm.4133 », le document Kaiser, Henry F. 1958. « The Varimax Criterion for Analytic Rotation in Factor Analysis. » Psychometrika 23 (3) : 187-200, accessible via le lien https://doi.org/10.1007/BF02289233 voire encore, le document Valle, Sergio, Weihua Li, and S. Joe Qin. 1999. « Selection of the Number of Principal Components: The Variance of the Reconstruction Error Criterion with a Comparison to Other Methods. » Industrial and Engineering Chemistry Research 38 (11): 4389-4401, accessible par le lien https://doi.org/ 10.1021/ie990110i). Ces publications présentent des méthodes basées sur les valeurs propres des composantes principales s'appuyant sur des critères de Malinowski, Nelson et Median et appliquées au débruitage de données d'imagerie par résonance magnétique CEST in vivo.

D'autres méthodes ont été proposées pour le débruitage des données expérimentales d'IRM de diffusion. Certaines de ces méthodes sont basées sur la théorie des matrices aléatoires comme le divulgue par exemple le document Veraart, Jelle, Dmitry S. Novikov, Daan Christiaens, Benjamin Ades-aron, Jan Sijbers, and Els Fieremans - 2016 - « Denoising of Diffusion MRI Using Random Matrix Theory. » NeuroImage 142: 394-406, accessible via le lien « https://doi.org/ 10.1016/j.neuroimage.2016.08.016 ». D'autres méthodes sont basées sur le rétrécissement de la valeur singulière comme le décrivent le document Ma, Xiaodong,

Kâmil Uğurbil, and Xiaoping Wu - 2020 - « Denoise Magnitude Diffusion Magnetic Resonance Images via Variance-Stabilizing Transformation and Optimal Singular-Value Manipulation.» NeuroImage 215 (July): 116852, accesible via le lien « https://doi.org/10.1016/j. neuroimage.2020.116852 » dont les principes mathématiques sont expliqués dans le document Gavish, Matan, and David L. Donoho - 2017 - « Optimal Shrinkage of Singular Values. » IEEE Transactions on Information Theory 63 (4): 2137-52, accessible via le lien « https:// doi.org/10.1109/TIT.2017.2653801 »).

A l'instar du procédé connu illustré par la figure 5, la plupart des méthodes proposées dans la littérature se basent sur les valeurs propres $\Lambda$ pour déterminer les k composantes à préserver et celles à exclure lors de la reconstruction des données. Ainsi, comme le suggère la figure 5, une étape 130 consiste à comparer, en l'espèce via une échelle logarithmique, les valeurs propres $\lambda_j$ des q composantes principales, j étant compris entre 1 et q.

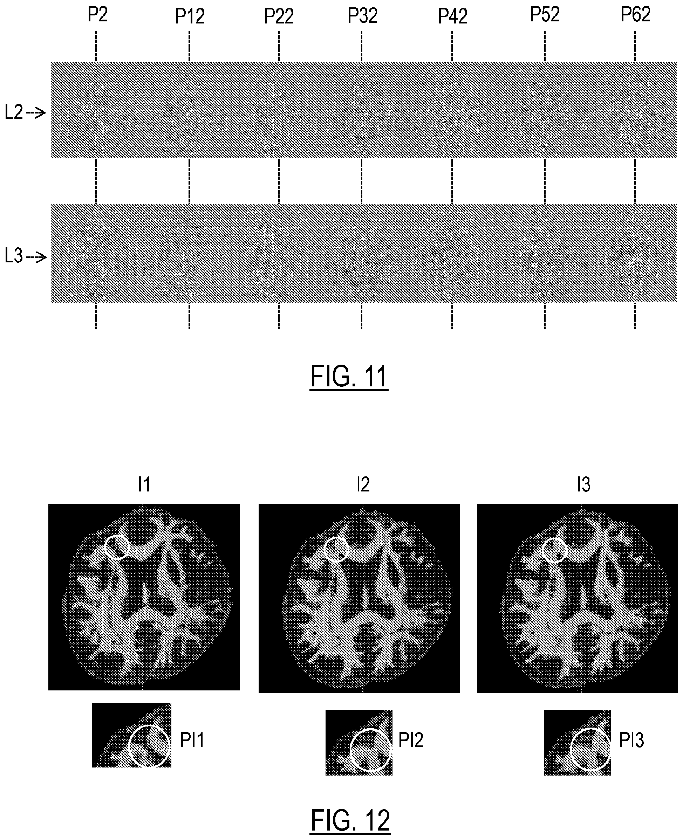

En dessous d'un certain seuil prédéterminé de variance, les composantes principales sont considérées comme exprimant peu ou pas d'informations pertinentes et sont rejetées. Seules les k premières composantes principales, c'est-à-dire celles dont les variances ou valeurs propres sont supérieures audit seuil sont conservées pour être exploitées à l'étape 140. Ces méthodes présentent un inconvénient majeur car elles conduisent généralement à ignorer des structures anatomiques et des informations pathologiques cachées dans certaines composantes principales écartées. Cet inconvénient découle directement de l'utilisation, comme principal sélecteur, des valeurs propres qui sont des mesures de variance des différentes composantes principales, mesurant aveuglément le bruit et les informations pertinentes. L'analyse en composantes principales part généralement du principe selon lequel la variance indique la présence ou l'absence d'informations importantes, ce qui suppose que les données expérimentales analysées ont un rapport signal sur bruit élevé, c'est-à-dire que la puissance du signal d'intérêt est plus forte que la puissance du bruit. Il s'agit d'une hypothèse forte et parfois incorrecte, en particulier pour des données expérimentales d'imagerie par résonance magnétique particulièrement bruitées. Une telle situation survient, par exemple, lorsque le niveau de bruit dans les données expérimentales délivrées par un appareil d'imagerie par résonance magnétique est élevé ou lorsque les signaux d'intérêt concernent une zone anatomique ou pathologique réduite au regard de la taille des images considérées. La plupart des méthodes proposées dans la littérature, basées sur les valeurs propres, écartent ainsi des composantes principales contenant des structures anatomiques ou informations pathologiques d'intérêt cachées car les contributions de ces zones aux valeurs propres des composantes associées sont faibles. Ainsi, une quantité non négligeable d'informations pertinentes est perdue car amalgamée au bruit selon l'état de la technique. La sélection des composantes principales ne peut se faire selon de tels critères que nous pourrions qualifier de simplistes ou triviaux pour garantir une exploitation pertinente des données expérimentales et délivrer des indicateurs ou biomarqueurs fiables à partir de données expérimentales débruitées, comme nous pourrons le voir en lien avec la figure 12 à titre exemple.

L'invention répond aux inconvénients soulevés par l'état de la technique. Parmi les nombreux avantages procurés par l'invention, nous pouvons mentionner plus particulièrement qu'un procédé de débruitage selon l'invention comporte une étape de sélection des composantes principales permettant d'obtenir un meilleur compromis entre efficacité du débruitage et conservation de l'information spatiale pertinente des données expérimentales considérées lors de leur reconstruction pour produire des données expérimentales débruitées. A titre d'exemple, un critère de sélection proposé est basé sur la diminution de la variance d'une image de score associée à une composante principale après l'application d'un filtre de lissage sur celle-ci. Cette technique permet d'exploiter l'information spatiale cachée, notamment les structures anatomiques et les informations pathologiques, dans toutes les composantes principales extraites ou calculées et permet de sélectionner celles permettant d'exprimer des informations importantes, indépendamment les unes des autres et de leur ordonnancement découlant de leur extraction ou production, minimisant ainsi le risque de rejeter des composantes principales pertinentes. L'invention permet en outre de disposer d'une étape de filtrage que nous pouvons qualifier d'adaptatif, c'est-à-dire permettant de débruiter davantage les données expérimentales considérées, en rejetant le bruit spatial persistant lié à chaque composante principale extraite, tout en adaptant le degré de filtrage des composantes principales à la quantité d'informations pertinentes que chacune d'entre-elles permettent d'exprimer.

A cette fin, l'invention prévoit un procédé tel que défini par la revendication 1 d'atténuation du bruit de données expérimentales fruits d'acquisitions multiples par un appareil d'imagerie de résonance magnétique en lien avec un volume élémentaire d'intérêt, ci-après nommé « voxel » d'intérêt, parmi une pluralité de voxels, ledit procédé étant mis en œuvre par une unité de traitement d'un système d'analyse d'imagerie médicale. Un tel procédé comporte :

- une étape de collecte desdites données expérimentales en lien avec la pluralité de voxels ;
- une étape de production d'un ensemble ordonné, selon leurs valeurs propres respectives, de q composantes principales ainsi que leurs scores respectifs pour la pluralité de voxels, lesdits scores correspondant à la projection desdites données expérimentales sur lesdites q composantes principales ;
- une étape de sélection de k premières composantes principales parmi les q composantes principales produites ;
- une étape de projection desdites données expérimentales sur les k composantes principales sélectionnées et de production de données expérimentales atténuées en bruit en lien avec le volume élémentaire d'intérêt.

Pour optimiser le débruitage des données expérimentales sans perdre de l'information spatiale, l'étape de sélection de k composantes principales d'un tel procédé comporte :

- une sous-étape de production d'images des scores des composantes principales pour la pluralité de voxels et d'indicateurs informatifs quantifiant l'information spatiale contenue dans lesdites images des scores associés auxdites composantes principales sous la forme d'un taux de diminution des valeurs d'une caractéristique déterminée desdits scores avant et après l'application d'un filtre de lissage sur lesdits scores, ladite caractéristique déterminée des scores pour une pluralité de voxels appartenant à un ensemble de caractéristiques, prises seules ou en combinaison, comportant la variance, l'écart-type, la médiane et la moyenne ;
- une sous-étape de détermination du rang k pour sélectionner les k premières composantes principales à partir desdits indicateurs informatifs produits.

Selon un premier mode de réalisation avantageux, la sous-étape de détermination du rang k peut consister en le calcul de la différence mathématique entre des valeurs desdits indicateurs informatifs lorsque ces derniers sont ordonnés conformément aux composantes principales produites, le rang k étant celui de la composante principale pour laquelle ladite différence mathématique devient inférieure ou égale à un seuil déterminé.

En variante, ladite sous-étape de détermination du rang k peut consister en la détection d'un plateau décrit par les valeurs des indicateurs informatifs, depuis l'indicateur informatif de la composante principale de rang q vers l'indicateur informatif de la composante principale de rang 1, le rang k étant celui de la première composante principale dont la valeur de l'indicateur informatif s'écarte dudit plateau.

Pour renforcer le poids des composantes principales prépondérantes lors de la reconstruction des données expérimentales ainsi débruitées, l'étape de sélection de k composantes principales d'un procédé selon l'invention peut comporter une sous-étape d'application d'une opération de filtrage des k composantes principales sélectionnées, dont l'intensité de filtrage est spécifique à chacune des k composantes principales sélectionnées et proportionnelle à l'indicateur informatif de cette dernière.

Dans ce cas, l'opération de filtrage peut consister en l'exploitation seule ou combinée d'un filtre gaussien, d'un filtre moyen, d'un filtre médian, d'un filtre de diffusion anisotropique.

Selon un deuxième objet, l'invention concerne un produit programme d'ordinateur tel que défini par la revendication 7 comportant une ou plusieurs instructions de programme interprétables par l'unité de traitement d'un système d'analyse d'imagerie médicale, lesdites instructions de programme étant chargeables dans une mémoire non volatile dudit système d'analyse d'imagerie médicale, dont l'exécution desdites instructions par ladite unité de traitement provoque la mise en œuvre d'un procédé d'atténuation du bruit de données expérimentales selon l'invention.

Selon un troisième objet, l'invention concerne un support de mémorisation lisible par un ordinateur comportant les instructions d'un tel produit programme d'ordinateur selon ladite invention.

Selon un quatrième objet, l'invention concerne un système d'analyse d'imagerie médicale tel que défini par la revendication 9 comportant une unité de traitement agencée pour atténuer le bruit de données expérimentales fruits d'acquisitions multiples par un appareil d'imagerie de résonance magnétique en lien avec un volume élémentaire d'intérêt, ci-après nommé « voxel » d'intérêt, parmi une pluralité de voxels, ladite unité de traitement étant configurée pour :

- collecter desdites données expérimentales en lien avec la pluralité de voxels ;
- produire un ensemble ordonné, selon leurs valeurs propres respectives, de q composantes principales ainsi que leurs scores respectifs pour la pluralité de voxels, lesdits scores correspondant à la projection desdites données expérimentales sur lesdites q composantes principales;
- sélectionner k premières composantes principales parmi les q composantes principales produites ;
- projeter lesdites données expérimentales sur les k composantes principales sélectionnées et produire des données expérimentales atténuées en bruit en lien avec le volume élémentaire d'intérêt.

Pour optimiser le débruitage des données expérimentales sans perdre de l'information spatiale, l'unité de traitement d'un tel système est configurée pour :

- produire des images des scores des composantes principales pour la pluralité de voxels et des indicateurs informatifs quantifiant l'information spatiale contenue dans lesdites images des scores associés auxdites composantes principales sous la forme d'un taux de diminution des valeurs d'une caractéristique déterminée desdits scores avant et après l'application d'un filtre de lissage sur lesdits scores, ladite caractéristique déterminée des scores pour une pluralité de voxels appartenant à un ensemble de caractéristiques, prises seules ou en combinaison, comportant la variance, l'écart-type, la médiane et la moyenne ;
- sélectionner les k premières composantes principales à partir desdits indicateurs informatifs produits.

Selon un mode de réalisation avantageux, un tel système d'analyse d'imagerie médicale peut comporter une mémoire de programmes comportant les instructions de programme d'un produit programme d'ordinateur conforme à l'invention.

De plus, l'invention concerne un procédé tel que défini par la revendication 2 et un système d'analyse d'imagerie médicale tel que défini par la revendication 10, selon lesquels l'analyse en composantes principales est remplacée par une décomposition en valeurs singulières, en alternative au procédé de la revendication 1 et au système de la revendication 9, respectivement.

D'autres caractéristiques et avantages apparaîtront plus clairement à la lecture de la description qui suit et à l'examen des figures qui l'accompagnent parmi lesquelles :

- la figure 1, d'ores-et-déjà décrite, illustre une description simplifiée d'un système d'analyse d'images obtenues par résonance magnétique ;
- la figure 2, d'ores-et déjà-décrite, illustre une description simplifiée d'une variante d'un système d'analyse d'images obtenues par magnétique ;
- la figure 3, d'ores-et-déjà décrite, présente un exemple d'un spectre Z sous la forme d'un ensemble d'échantillons $Zi(\Delta\omega)$ selon des décalages fréquentiels relatifs $\Delta\omega$ par rapport à la fréquence de l'eau, de tels décalages $\Delta\omega$ étant exprimés en ppm ;
- la figure 4, d'ores-et-déjà décrite, illustre le principe de l'analyse en composantes principale consistant à transformer des variables ou données liées entre-elles (également qualifiées de « corrélées » en statistique) en nouvelles variables ou données décorrélées les unes des autres ;
- la figure 5, d'ores-et-déjà décrite, illustre un procédé connu pour débruiter un spectre Zi bruité découlant d'une acquisition CEST selon l'état de la technique ;
- la figure 6, d'ores-et-déjà décrite, illustrent les données expérimentales projetées sur différentes composantes principales extraites d'un ensemble de données CEST après remodelage dans leur espace d'origine ;
- la figure 7 illustre un exemple d'algorithme fonctionnel d'un procédé de débruitage selon l'invention ;
- la figure 8 illustre un exemple de calcul d'un indicateur informatif de composante principale propre à l'invention ;
- la figure 9 illustre un exemple de sélection de composantes principales selon l'invention ;
- la figure 10 illustre un exemple d'atténuation du bruit de données expérimentales de diffusion HARDI pour différentes phases ;
- la figure 11 illustre un exemple de bruit retiré par la mise en œuvre de l'invention de telles données HARDI pour différentes phases ;
- la figure 12 illustre un exemple d'exploitation et du bénéfice découlant d'une telle exploitation de données débruitées grâce à l'invention dans le domaine de la tractographie ;
- la figure 13 illustre un exemple d'atténuation du bruit de données expérimentales de perfusion pour différentes phases ;
- la figure 14 illustre un exemple de bruit retiré par la mise en œuvre de l'invention de telles données de perfusion pour différentes phases ;
- la figure 15 illustre un mode réalisation avantageux d'un procédé selon l'invention permettant un ajustement des poids propres aux différentes composantes principales sélectionnées préalablement à la projection des données expérimentales bruitées sur celles-ci.

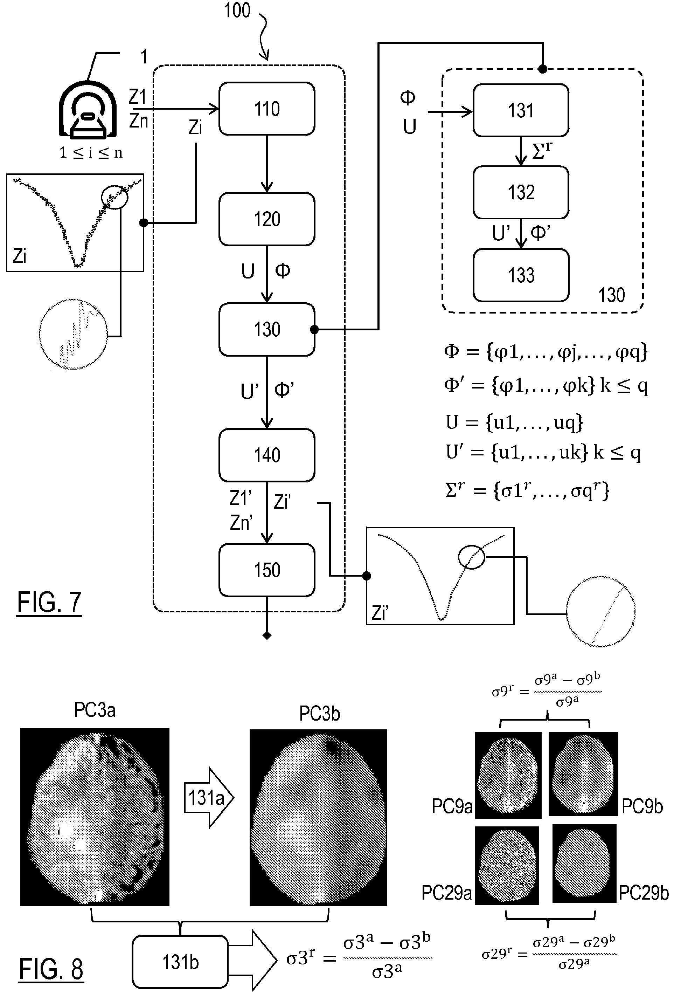

Un procédé 100 d'atténuation du bruit d'images médicales, conforme à l'invention et illustré par la figure 7, se traduit avantageusement sous la forme d'un produit programme d'ordinateur dont les instructions de programme sont destinées à être implantées dans la mémoire de programmes d'un élément d'un système d'imagerie médicale, tels que le système S selon les figures 1 et 2, par exemple un ordinateur ou un serveur informatique ou, plus généralement, de tout objet électronique disposant d'une puissance de calcul suffisante.

La figure 7 illustre ainsi un procédé 100, conforme à l'invention, d'atténuation du bruit de données expérimentales fruits d'acquisitions multiples par un appareil d'imagerie de résonance magnétique, tel que l'appareil 1 du système d'analyse médicale S illustré par les figures 1 et 2, en lien avec une pluralité de n voxels. Un tel procédé comporte certaines étapes communes avec le procédé 100 selon la figure 5. Ainsi, un procédé de « débruitage » 100 selon l'invention peut comporter si nécessaire, une étape 110 de collecte ou d'acquisition de données expérimentales bruitées en lien ou concernant ladite pluralité de voxels V1 à Vn. De telles données expérimentales peuvent consister en des courbes telles que des spectres Z1 à Zn à la suite d'une acquisition de type CEST à l'instar de l'exemple lié aux figures 3 et 5 ou, plus généralement, toutes données expérimentales pour un ensemble de voxels ou de pixels en lien avec un volume ou une tranche d'un organe concerné par une pluralité de m acquisitions selon différents instants ou différentes phases par exemple. De telles données expérimentales pourraient ainsi, de manière non exhaustive, résulter d'une acquisition multiphases d'imagerie de diffusion à haute résolution angulaire HARDI, voire concerner le domaine de l'imagerie de perfusion.

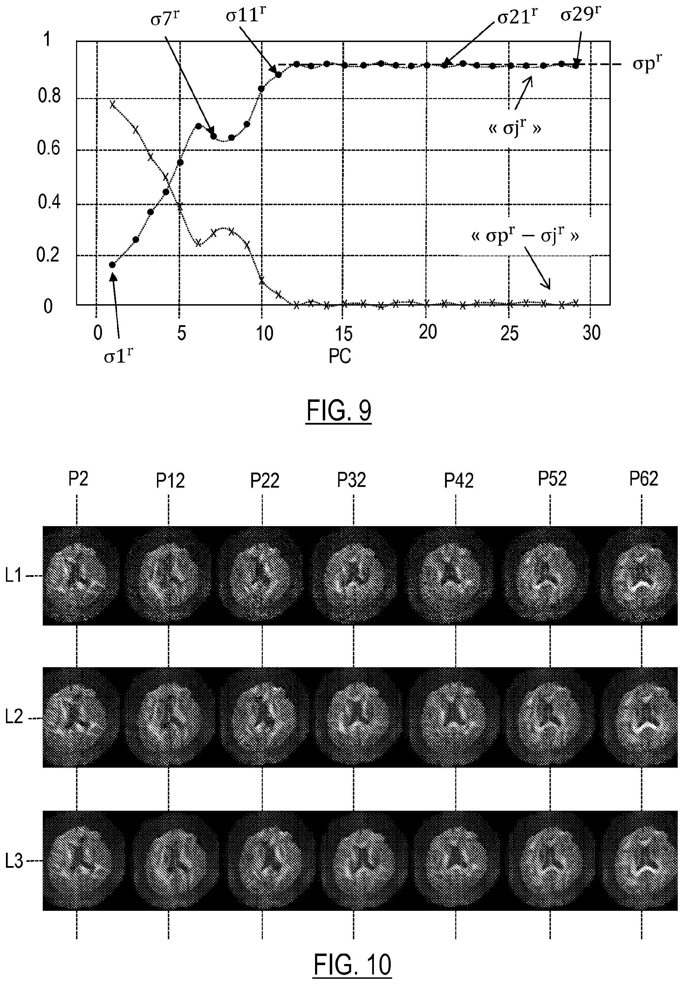

A titre d'exemple préféré mais non limitatif, la figure 7 décrit, à l'instar de la figure 5, des données expérimentales Zi liées à un voxel d'intérêt parmi les n voxels acquis. La figure 7 illustre des données expérimentales Zi, sous la forme d'un spectre bruité identique à celui illustré en figure 5. Un tel bruit se traduit par des discontinuités ou oscillations plus ou moins marquées selon les décalages fréquentiels concernés comme en atteste l'agrandissement partiel dudit spectre Zi. Pour atténuer ce phénomène, à l'instar du procédé 100 connu et illustré par la figure 5, un procédé 100 conforme à l'invention comporte une étape 120 de mise en œuvre d'une analyse en composantes principales pour produire ou calculer q composantes principales, ladite étape étant similaire à l'étape 120 du procédé connu 100 selon la figure 5. Une telle étape 120 consiste à déterminer q composantes principales symbolisées ou associées respectivement à des valeurs propres $\lambda 1$, ..., $\lambda q$ ou à des valeurs singulières $s_1$ à $s_q$ formant un ensemble ordonné de composantes principales dont le rang est déterminé selon un ensemble de scalaires de valeurs décroissantes, tel que les valeurs propres $\Lambda = \{\lambda 1, ..., \lambda q\}$ ou les valeurs singulières S. Un tel procédé 100 selon l'invention comporte en outre une étape 130 de détermination ou de sélection de k composantes principales parmi les q produites, soit les composantes principales de rangs 1 à k pour déterminer un ensemble plus restreint de composantes principales. La sélection 130, conforme à l'invention et illustrée par la figure 7, se distingue clairement de l'approche décrite très majoritairement dans l'état de la technique se fondant sur les valeurs propres $\Lambda$. Une telle étape 130 propre à l'invention sera détaillée ultérieurement. Un procédé 100 selon l'invention comporte en outre, à l'instar des procédé 100 connus tels qu'illustrés par la figure 5, une étape 140 de projection des données expérimentales bruitées Zi sur les k composantes principales sélectionnées à l'étape 130 et de production de données expérimentales atténuées en bruit Zi' ou « débruitées » en lien avec un volume élémentaire d'intérêt. Nous pourrons constater l'efficacité accrue d'un procédé 100 selon l'invention grâce à une meilleure sélection, voire une modification des k composantes principales, préalablement à ladite projection 140 en examinant le spectre Zi' débruité décrivant une courbe particulièrement lisse au regard de celle décrite par le spectre d'origine Zi. A l'instar de celui-ci illustré par la figure 5, l'étape 140 du procédé 100 selon la figure consiste à projeter l'ensemble des données expérimentales Z1, ...,Zi, ..., Zn bruitées sur les k composantes principales sélectionnées et produire des données expérimentales atténuées en bruit Z1', ...,Zi', ..., Zn' pour les voxels d'intérêt V1 à Vn. Ainsi, de manière optionnelle et avantageuse, un tel procédé 100 selon l'invention peut comporter une étape 150 d'exploitation conjointe des données expérimentales débruitées Z1', ..., Zi', ..., Zn' pour tout ou partie de pluralité desdits voxels V1 à Vn d'intérêt. Une telle exploitation peut par exemple consister en le dénombrement de fibres en tractographie à partir de données expérimentales de diffusion HARDI comme nous l'étudierons en lien avec les figures 10 à 12.

L'invention se distingue donc principalement par la mise en œuvre de l'étape 130 permettant de sélectionner les composantes principales pertinentes, c'est-à-dire, permettant d'optimiser l'atténuation du bruit sans pour autant perdre d'informations spatiales pertinentes. Ainsi, comme l'illustre la figure 7, une telle étape 130 comporte une sous-étape 131 de production d'indicateurs informatifs, référencés $\sigma 1^r$, ..., $\sigma q^r$ sur la figure 7, respectivement calculés pour les q composantes principales produites de rangs 1 à q. Un tel ensemble $\Sigma^r$ d'indicateurs informatifs $\sigma 1^r$ à $\sigma q^r$ est une alternative à l'ensemble $\Lambda$ des valeurs propres $\lambda 1$ à $\lambda q$ exploité directement par les procédés connus pour sélectionner k composantes principales parmi les q extraites. Un tel indicateur informatif $\sigma j^r$ caractérise la capacité d'une composante principale de rang j à exprimer de l'information spatiale pertinente. Il peut consister avantageusement en un taux de diminution des valeurs d'une caractéristique déterminée des scores de ladite composante principale en lien avec la pluralité de voxels V1 à Vn considérés ou d'intérêt, avant et après l'application d'un filtre de lissage sur lesdites scores. Une telle opération 131a est illustrée par la figure 8. Celle-ci décrit pour la composante principale de rang 3 deux images PC3a et PC3b illustrant les scores de ladite composante principale de rang 3 pour l'ensemble des voxels considérés. L'image PC3a décrit lesdits scores avant l'application 131a d'un filtre de lissage et l'image PC3b décrit ces mêmes scores après ladite application 131a du filtre de lissage. Cette dernière apparaît ainsi davantage floue au regard de l'image PC3a sur la figure 8. Une telle opération 131a peut avantageusement consister en l'application d'un filtre gaussien d'un facteur F de valeur prédéterminée, éventuellement paramétrable.

A titre d'exemple préféré mais non limitatif, ladite caractéristique déterminée peut consister en l'écart-type desdits scores. L'indicateur informatif $\sigma j^r$ d'une composante principale de rang j peut alors être calculé en une opération 131b telle que :

$$\sigma j^r = \frac{\sigma j^a - \sigma j^b}{\sigma j^a}$$

où $\sigma j^a$ est l'écart-type des scores de la composantes principale de rang j avant l'application 131a du filtre de lissage et $\sigma j^b$ est l'écart-type desdits scores après l'application 131a dudit du filtre de lissage.

En l'espèce sur la figure 8, l'indicateur informatif $\sigma 3^r$ de la composante principale de rang 3 est tel que :

$$\sigma 3^r = \frac{\sigma 3^a - \sigma 3^b}{\sigma 3^a}$$

De la même manière, la figure 8 illustre le calcul de l'indicateur informatif $\sigma 9^r$ de la composante principale de rang 9 est tel que :

$$\sigma 9^r = \frac{\sigma 9^a - \sigma 9^b}{\sigma 9^a}$$

ou encore de l'indicateur informatif $\sigma 29^r$ de la composante principale de rang 29 est tel que :

$$\sigma 29^r = \frac{\sigma 29^a - \sigma 29^b}{\sigma 29^a}$$

De cette manière, l'ensemble $\Sigma r$ des indicateurs informatifs des q composantes principales de rangs 1 à q produites à l'étape 120 et ordonnées selon leurs valeurs propres $\Lambda$ ou valeurs singulières S respectives, peut être constitué à l'issue de la mise en œuvre de la sous-étape 131. En variante, cet ensemble $\Sigma r$ des indicateurs informatifs des q composantes principales peut être ordonné selon les valeurs numériques desdits indicateurs informatifs.

En variante ou en complément, une telle caractéristique déterminée $\sigma j r$ pourrait exploiter, en lieu et place de l'écart-type, la variance, l'entropie, la médiane ou encore la moyenne desdits scores de la composante principale de rang j, voire résulter d'une combinaison de tout ou partie de celles-ci.

Une fois l'ensemble $\Sigma^r$ des indicateurs informatifs calculé, l'étape 131 d'un procédé 100 selon l'invention comporte une sous-étape 132 de détermination du rang k pour sélectionner les k premières composantes principales (symbolisées par l'ensemble des vecteurs propres $\Phi'=\{\varphi 1, ..., \varphi k\}$ ou la matrice $U'=\{u_1, ..., u_k\}$ sur la figure 7) à partir dudit ensemble $\Sigma^r$ desdits indicateurs informatifs. La mise en œuvre d'une telle sous-étape 132 est illustrée par la figure 9. Cette dernière présente, en liaison avec les q=29 composantes principales de rangs 1 à 29 dont les scores PC1 à PC29 pour une pluralité de voxels d'intérêt sont illustrés par la figure 6, l'ensemble $\Sigma^r$ des indicateurs informatifs calculés en 131b et ordonnés selon les rangs respectifs des q=29 composantes principales produites à l'étape 120. Les valeurs desdits indicateurs informatifs décrivent une courbe « $\sigma j^r$ » sensiblement croissante lorsque le rang des composantes principales croît pour atteindre un plateau $\sigma p^r$ de l'ordre de quatre-vingt-dix pourcents sur l'exemple illustré par la figure 9 à partir du rang 12. La valeur de convergence ou d'un plateau $\sigma p^r$ dépend de la force du lissage qui a été appliqué aux images de scores de la figure 8 et de la caractéristique utilisée pour produire les indicateurs informatifs. Comme observé précédemment en liaison avec la figure 6, la composante de rang j=1 semble exprimer davantage d'informations spatiales que les composantes de rangs j supérieurs à 10. L'étape 132 permet ainsi, selon différents modes de réalisation, d'objectiver la sélection des k premières composantes principales pour obtenir le compromis recherché. Ainsi, selon un premier mode de réalisation, une telle sous-étape 132 peut consister en le calcul de la différence mathématique entre des valeurs desdits indicateurs informatifs $\Sigma^r = \{\sigma 1^r, ..., \sigma q^r\}$ lorsque ces derniers sont ordonnés conformément aux rangs j des q composantes principales produites. Le rang k peut être déterminé à partir d'une telle différence entre deux indicateurs informatifs de rangs consécutifs. Ainsi, dès que ladite différence devient inférieure ou égale (en valeur absolue) à un seuil déterminé, par exemple un seuil d'une valeur inférieure à trois centièmes, le rang k recherché est celui de la composante principale de rang immédiatement inférieur à celui de la composante principale pour laquelle l'indicateur informatif est sensiblement égal (différence sensiblement nulle ou inférieure audit seuil) à celui de la composante principale de rang qui lui est immédiatement supérieur. Une telle approche est pertinente lorsque les indicateurs informatifs, ordonnés selon les rangs des composantes principales qui leur sont respectivement associées, décrivent une courbe « $\sigma j^r$ » qui est sensiblement croissante jusqu'à atteindre un plateau $\sigma p^r$. En variante ou en complément du calcul d'une différence mathématique entre deux indicateurs informatifs de rangs consécutifs, l'invention prévoit que l'on puisse calculer une différence mathématique moyenne entre une collection accrue d'indicateurs consécutifs, une différence entre les indicateurs informatifs de rangs inférieur(s) et/ou supérieur(s) à un indicateur infirmatif donné ou toute autre technique équivalente. Toutefois, comme l'indique la courbe « σjr » illustrée en figure 9, celle-ci peut présenter un ou plusieurs paliers. En l'espèce, un premier palier est décrit par les valeurs des indicateurs informatifs σ7r et σ8r des composantes principales de rangs 7 et 8. Un second palier est décrit par les valeurs des indicateurs informatifs σ12r à σ29r des composantes principales de rangs supérieurs ou égaux à 12. Pour ne pas déterminer un rang k trop faible lié à l'existence d'un palier intermédiaire pour lequel la dérivée de la courbe « σjr » est sensiblement nulle, un tel calcul de la dérivée peut être assorti ou combiné avec le calcul d'un écart (décrit par la courbe « σpr- σjr » sur la figure 9) avec l'indicateur informatif associé à la composante principale de rang le plus élevé en l'espèce le rang q=29, voire l'indicateur informatif de valeur maximale. Si ledit écart « σpr- σjr » est très faible (par exemple inférieur à trois centièmes), alors l'étape 132 considère que la courbe a atteint l'asymptote ou le plateau σpr. Le rang k est ainsi déterminé comme étant celui-ci de la composante principale immédiatement de rang inférieur. C'est par exemple le cas de la composante de rang k=11 qui est la dernière présentant un indicateur informatif σ11r « échappant » au plateau σpr ou, plus précisément, dont la valeur s'écarte substantiellement de la valeur σpr dudit plateau. En revanche, si ledit écart « σpr- σjr » est conséquent, par exemple supérieur à dix pourcents de la valeur σpr, (cas de la composante principale de rang 7 dont l'indicateur informatif σ7r est sensiblement égal à celui σ8r de la composante principale de rang immédiatement supérieur sur l'exemple illustré par la figure 9), alors un tel palier de la courbe « σjr » est ignoré.

En variante, une telle sous-étape 132 peut consister en la détection d'un plateau σpr, tel évoqué précédemment, décrit par les valeurs des indicateurs informatifs σjr (j étant compris entre 1 et q) en parcourant ces dernières depuis le rang le plus élevé des composantes principales, en l'espèce le rang q=29, vers le rang 1. Par hypothèse, un tel plateau σpr existe pour les composantes principales des rangs les plus élevés, en l'espèce pour la figure 9, le plateau σpr est de l'ordre de quatre-vingt-dix pourcents. Le rang k recherché est déterminé comme étant celui de la première composante principale dont la valeur de l'indicateur informatif σkr s'écarte dudit plateau σpr de manière significative (c'est-à-dire au-delà d'un seuil prédéterminé, par exemple cinq pourcents de la valeur dudit plateau). En l'espèce, l'indicateur informatif σ11r est le premier à décrire une telle valeur suffisamment distincte de la valeur dudit plateau σpr. Le rang k recherché est donc celui de ladite composante principale de k égal à onze.

L'invention ne saurait être limitée à de telles opérations mises en œuvre dans le cadre de la sous-étape 132 pour détecter la valeur du rang k à partir de l'ensemble ordonné des indicateurs informatifs Σr.

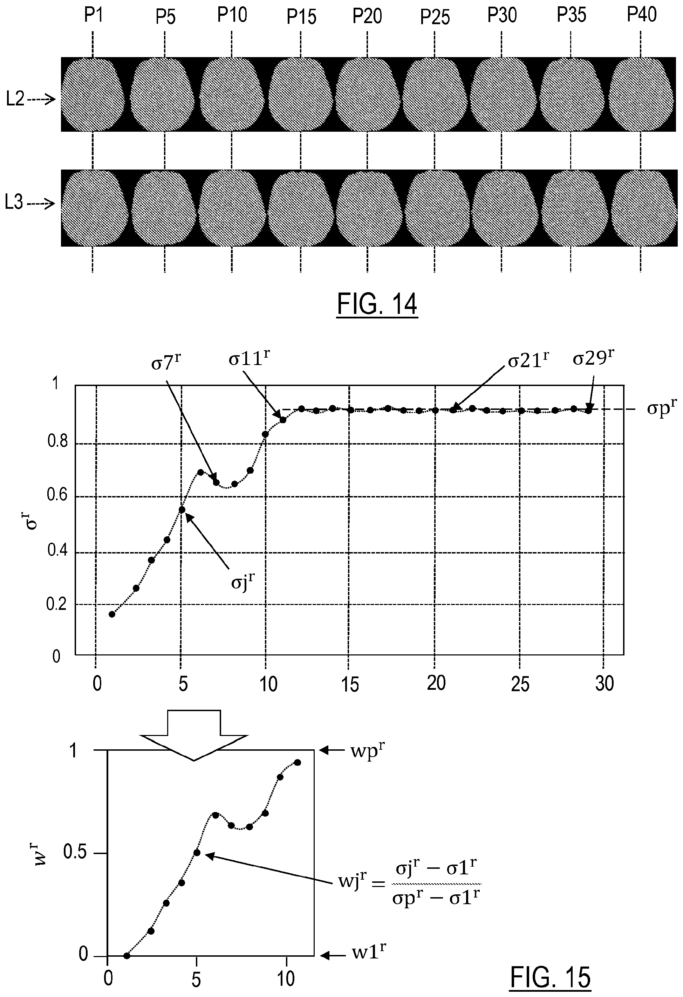

L'invention prévoit en outre de pouvoir modifier les k composantes principales ainsi sélectionnées à l'étape 130 (symbolisées par le vecteur Φ' en figure 7), préalablement à la mise en œuvre de l'étape 140. L'objectif est d'accroître les contributions des composantes principales les plus « informatives » (dont les rangs sont les plus faibles) au regard de celles dont les capacités respectives à exprimer des informations spatiales sont plus faibles, celles de rangs plus élevés. Pour cela, une telle étape 130 peut comporter en outre une sous-étape 133 d'application d'une opération de filtrage des k composantes principales sélectionnées, dont l'intensité de filtrage est spécifique à chacune des k composantes principales de rangs 1 à k inférieur ou égal à q) sélectionnées et proportionnelle à l'indicateur informatif σ1r à σkr de cette dernière. Une telle opération de filtrage 133 peut consister en l'exploitation seule ou combinée d'un filtre gaussien, d'un filtre moyen, d'un filtre médian, d'un filtre de diffusion anisotropique, d'un filtre d'ondelette ou « wavelet » selon une terminologie anglo-saxonne ou tout filtre, spatial ou autre, pouvant être exploité dans ce contexte. A titre d'exemple préféré mais non limitatif, une telle sous-étape 133 peut consister en l'ajustement de poids wjr propres aux composantes principales de rangs j réalisé à partir des valeurs σjr. Les poids, dans cet exemple, peuvent être multipliés linéairement par la force A d'un filtre gaussien. Chaque composante sélectionnée, c'est-à-dire de rang j, j étant inférieur ou égal à k, est filtrée par un facteur de wjr x A. En variante, ou complément de la multiplication linéaire, les poids peuvent être préalablement exprimés sous la forme d'entrées d'une fonction f logarithmique, exponentielle ou polynomiale. De cette manière, chaque image de score PCj sélectionnée, c'est-à-dire de rang j, j étant inférieur ou égal à k, peut être filtrée par un facteur défini par f(wjr) x A. Enfin, les images de scores « filtrées » sont utilisées pour reproduire la matrice de scores U. Comme l'illustre la figure 15, un tel poids wjr peut être calculé à partir de la courbe « σjr » illustrée en figure 9, comme suit :

$$ wj^r = \frac{\sigma j^r - \sigma 1^r}{\sigma p^r - \sigma 1^r} $$

où σjr est la valeur de l'indicateur informatif de la composante principale de rang j, σ1r est la valeur de l'indicateur informatif de la composante principale de rang 1 et σpr la valeur du plateau de la courbe « σjr » décrite par lesdits indicateurs informatifs Σr. Ainsi, un tel poids wjr est compris entre w1r=0 et wpr =1 (c'est-à-dire lorsque la courbe « σjr » a atteint un plateau σpr).

L'invention ne saurait être limitée à ces seuls exemples de calculs des poids wjr de la sous-étape 133.

Les figures 10 à 12 permettent d'illustrer le bénéfice découlant de la mise en œuvre d'un procédé pour atténuer le bruit de données expérimentales fruits d'acquisitions multiples par un appareil d'imagerie de résonance magnétique dans le domaine de l'imagerie de diffusion HARDI. Une telle acquisition de données expérimentales peut ainsi porter sur une pluralité de tranches, en l'espèce un cerveau humain, pour une pluralité de phases. La figure 10 illustre ainsi les données brutes, pour une tranche donnée, des phases deux, douze, vingt-deux, trente-deux, quarante-deux, cinquante-deux et soixante-deux, respectivement référencée P2, P12, P22, P32, P42, P52 et P62 sur la figure 10. Sur la première série d'images référencée L1, apparaissent les données expérimentales brutes, donc bruitées pour une pluralité de voxels et respectivement pour les différentes phases P2 à P62 évoquées précédemment. Les deuxième et troisième séries d'images L2 et L3 illustrent lesdites données expérimentales débruitées par la mise en œuvre de l'invention, selon que les k composantes principales sélectionnées aient fait l'objet d'un filtrage (étape 133 d'un procédé selon la figure 7) préalable (L3) ou non (L2) à la projection des données expérimentales sur lesdites k composantes principales. Nous pouvons visuellement constater le gain croissant apporté par l'invention depuis la série L1 vers la série L3.

De plus, les performances d'un procédé 100 selon l'invention peuvent être illustrées par la figure 11 qui décrit le bruit retiré à l'étape 140 d'un procédé selon l'invention, pour les différentes phases référencées P2, P12, P22, P32, P42, P52 et P62 sur la figure 10, en lien avec les données expérimentales débruitées des séries L2 et L3 de la figure 10. Il est manifeste qu'un tel bruit ne révèle aucune information spatiale d'intérêt.

La figure 12 décrit l'exploitation de données de diffusion HARDI décrites en lien avec la figure 10 dans le cadre d'une tractographie. La figure 12 présente trois images I1, I2, I3 en couleurs, traduites en niveau de gris, pour une tranche donnée, ainsi qu'un agrandissement partiel PI1, PI2 et PI3 de chaque image I1, I2, I3. Ces dernières ont été produites en une étape 150 à partir des données expérimentales respectivement brutes (série L1 sur la figure 10), de données débruitées par la mise en œuvre de l'invention et telles qu'illustrées par la série L2 sur la figure 10 et par la série L3 sur ladite figure 10. Lesdites agrandissements PI1, PI2 et PI3 permettant de voir, à l'œil nu, une mise en exergue croissante de fibres. Sur l'agrandissement PI1, une zone (cerclée de blanc) de l'organe pourrait être considérée comme morte, ce qui n'est pas le cas sur les agrandissements PI2 et PI3. En outre une telle exploitation permet de dénombrer de telles fibres. A partir des données brutes de la série L1 de la figure 10, il est possible de dénombrer de l'ordre de vingt-sept mille fibres. Elle sont de l'ordre de trente-quatre mille si des données débruitées formant la série L2 de la figure 10 sont exploitées et supérieures à trente-sept mille si les données débruitées formant la série L3 de la figure 10 sont exploitées. Un débruitage de données expérimentales selon l'invention apporte ainsi un gain particulièrement significatif.

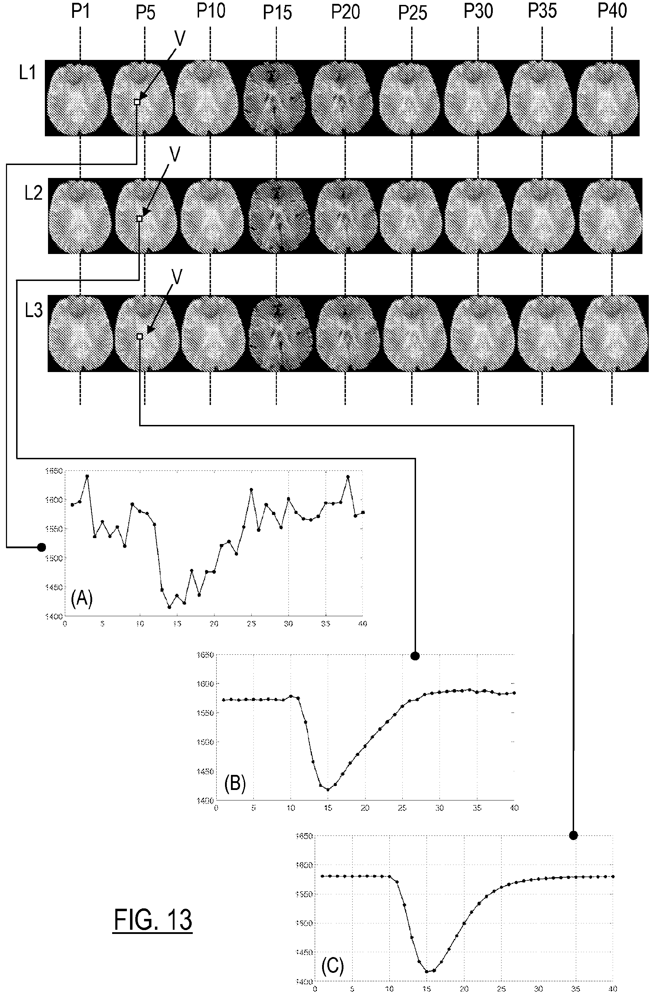

Les figures 13 et 14 illustrent, à l'instar des figures 10 et 11, le gain apporté par l'invention à partie de données de perfusion. Ainsi, l'acquisition de données expérimentales porte sur une pluralité de tranches, en l'espèce un cerveau humain, pour une pluralité de phases. La figure 13 illustre ainsi les données brutes, pour une tranche donnée, des phases une, cinq, dix, quinze, vingt, vingt-cinq, trente, trente-cinq et quarante, respectivement référencée P1, P5, P10, P15, P20, P25, P30, P35 et P40 sur la figure 13. Sur la première série d'images référencée L1, apparaissent les données expérimentales brutes, donc bruitées pour une pluralité de voxels et respectivement pour les différentes phases P1 à P40 évoquées précédemment. Les deuxième et troisième séries d'images L2 et L3 illustrent lesdites données expérimentales débruitées par la mise en œuvre de l'invention, selon que les k composantes principales sélectionnées ait fait l'objet d'un filtrage (étape 133 d'un procédé selon la figure 7) préalable (L3) ou non (L2) à la projection des données expérimentales sur lesdites k composantes principales. Nous pouvons visuellement constater le gain croissant apporté par l'invention depuis la série L1 vers la série L3. Ce gain est d'autant plus notable, lorsque l'on examine sur ladite figure 13, le spectre des données expérimentales, pour un voxel V d'intérêt, respectivement brutes (A), débruitées par la mise en œuvre de l'invention lorsque les k composantes principales sélectionnées n'ont pas fait l'objet d'un filtrage (B) et lorsque ledit filtrage a été réalisé (C). Nous pouvons remarquer que les courbes décrites respectivement par lesdits spectres sont de plus en plus lisses.

De plus, les performances d'un procédé 100 selon l'invention peuvent être illustrées par la figure 14 qui décrit le bruit retiré en lien avec les séries L2 et L3 de la figure 13. Il est manifeste qu'un tel bruit ne révèle aucune information spatiale d'intérêt.

## Revendications

1. Procédé (100) pour atténuer le bruit de données expérimentales (Zi) fruits d'acquisitions multiples par un appareil d'imagerie de résonance magnétique (1) en lien avec un volume élémentaire d'intérêt, ci-après nommé « voxel » d'intérêt, parmi une pluralité de voxels, ledit procédé (100) étant mis en œuvre par une unité de traitement (4) d'un système d'analyse d'imagerie médicale (S), ledit procédé (100) comportant :

   - une étape de collecte (110) desdites données expérimentales (Z1, ..., Zi, ..., Zn) en lien avec la pluralité de voxels (V1, ..., Vn) ;
   - une étape (120) de production d'un ensemble ordonné, selon leurs valeurs propres respectives, de q composantes principales, ainsi que leurs scores respectifs pour la pluralité de voxels (V1, ..., Vn), lesdits scores correspondant à la projection desdites données expérimentales sur lesdites q composantes principales ;
   - une étape de sélection (130) des k premières composantes principales parmi les q composantes principales produites ;

- une étape de projection (140) desdites données expérimentales (Zi) sur les k composantes principales sélectionnées et de production de données expérimentales atténuées en bruit (Zi') en lien avec le volume élémentaire d'intérêt;

ledit procédé étant **caractérisé en ce que** l'étape (130) de sélection de k composantes principales (130) dudit procédé (100) comporte :

- une sous-étape (131) de production d'images des scores des composantes principales pour la pluralité de voxels (V1, ..., Vn) et d'indicateurs informatifs ($\sigma 1^r$, ..., $\sigma q^r$) quantifiant l'information spatiale contenue dans lesdites images des scores associés auxdites composantes principales sous la forme d'un taux de diminution des valeurs d'une caractéristique déterminée desdits scores avant et après l'application d'un filtre de lissage sur lesdits scores, ladite caractéristique déterminée des scores pour une pluralité de voxels (V1, ..., Vn) appartenant à un ensemble de caractéristiques, prises seules ou en combinaison, comportant la variance, l'écart-type, la médiane et la moyenne ;
- une sous-étape (132) de détermination du rang k pour sélectionner les k premières composantes principales à partir desdits indicateurs informatifs ($\sigma 1^r$, ..., $\sigma q^r$) produits.

**2.** Procédé (100) pour atténuer le bruit de données expérimentales (Zi) fruits d'acquisitions multiples par un appareil d'imagerie de résonance magnétique (1) en lien avec un volume élémentaire d'intérêt, ci-après nommé « voxel » d'intérêt, parmi une pluralité de voxels, ledit procédé (100) étant mis en œuvre par une unité de traitement (4) d'un système d'analyse d'imagerie médicale (S), ledit procédé (100) comportant :

- une étape de collecte (110) desdites données expérimentales (Z1, ..., Zi, ..., Zn) en lien avec la pluralité de voxels (V1, ..., Vn) ;
- une étape (120) de production d'un ensemble ordonné, selon leurs valeurs singulières respectives, de q composantes principales, ainsi que leurs scores respectifs pour la pluralité de voxels (V1, ..., Vn), lesdits scores correspondant à la projection desdites données expérimentales sur lesdites q composantes principales ;
- une étape de sélection (130) des k premières composantes principales parmi les q composantes principales produites ;
- une étape de projection (140) desdites données expérimentales (Zi) sur les k composantes principales sélectionnées et de production de données expérimentales atténuées en bruit (Zi') en lien avec le volume élémentaire d'intérêt;

ledit procédé étant **caractérisé en ce que** l'étape (130) de sélection de k composantes principales (130) dudit procédé (100) comporte :

- une sous-étape (131) de production d'images des scores des composantes principales pour la pluralité de voxels (V1, ..., Vn) et d'indicateurs informatifs ($\sigma 1r$, ..., $\sigma qr$) quantifiant l'information spatiale contenue dans lesdites images des scores associés auxdites composantes principales sous la forme d'un taux de diminution des valeurs d'une caractéristique déterminée desdits scores avant et après l'application d'un filtre de lissage sur lesdits scores, ladite caractéristique déterminée des scores pour une pluralité de voxels (V1, ..., Vn) appartenant à un ensemble de caractéristiques, prises seules ou en combinaison, comportant la variance, l'écart-type, la médiane et la moyenne ;
- une sous-étape (132) de détermination du rang k pour sélectionner les k premières composantes principales à partir desdits indicateurs informatifs ($\sigma 1r$, ..., $\sigma qr$) produits.

**3.** Procédé (100) selon la revendication 1 ou 2 pour lequel la sous-étape (132) de détermination du rang k consiste en le calcul de la différence mathématique entre des valeurs desdits indicateurs informatifs ($\sigma 1^r$, ..., $\sigma q^r$) lorsque ces derniers sont ordonnés conformément aux q composantes principales produites, le rang k étant celui de la composante principale pour laquelle ladite différence mathématique devient inférieure ou égale à un seuil déterminé.

**4.** Procédé (100) selon la revendication 1 ou 2 pour lequel la sous-étape (132) de détermination du rang k consiste en la détection d'un plateau ($\sigma p^r$) décrit par les valeurs des indicateurs informatifs ($\sigma 1^r$, ..., $\sigma q^r$) lorsque ces derniers sont ordonnés conformément aux q composantes principales produites, depuis l'indicateur informatif ($\sigma q^r$) de la composante principale de rang q vers l'indicateur informatif ($\sigma 1^r$) de la composante principale de rang 1, le rang k étant celui de la première composante principale dont la valeur de l'indicateur informatif ($\sigma k^r$) s'écarte dudit plateau ($\sigma p^r$).

**5.** Procédé (100) selon l'une quelconque des revendications précédentes, comportant une sous-étape (133) d'application d'une opération de filtrage des k composantes principales sélectionnées, dont l'intensité de filtrage est spécifique à chacune des k composantes principales sélectionnées et proportionnelle à l'indicateur informatif ($\sigma 1^r$, ..., $\sigma 11^r$) de cette dernière.

**6.** Procédé (100) selon la revendication précédente, pour laquelle l'opération de filtrage (133) consiste en l'exploitation seule ou combinée d'un filtre gaussien, d'un filtre moyen, d'un filtre médian, d'un filtre de diffusion anisotropique.

7. Produit programme d'ordinateur comportant une ou plusieurs instructions de programme interprétables par l'unité de traitement (4) d'un système d'analyse d'imagerie médicale (S), lesdites instructions de programme étant chargeables dans une mémoire non volatile dudit système d'analyse d'imagerie médicale (S), **caractérisé en ce que** l'exécution desdites instructions par ladite unité de traitement (4) provoque la mise en œuvre d'un procédé (100) selon l'une quelconque des revendications précédentes.

8. Support de mémorisation lisible par un ordinateur comportant les instructions d'un produit programme d'ordinateur selon la revendication précédente.

9. Système (S) d'analyse d'imagerie médicale (S) comportant une unité de traitement (4) agencée pour atténuer le bruit de données expérimentales (Zi) fruits d'acquisitions multiples par un appareil d'imagerie de résonance magnétique (1) en lien avec un volume élémentaire d'intérêt, ci-après nommé « voxel » d'intérêt, parmi une pluralité de voxels, ladite unité de traitement (4) étant configurée pour :

   - collecter desdites données expérimentales (Z1, ..., Zi, ..., Zn) en lien avec la pluralité de voxels (V1, ..., Vn) ;
   - produire un ensemble ordonné, selon leurs valeurs propres respectives, de q composantes principales ainsi que leurs scores respectifs pour la pluralité de voxels (V1, ..., Vn), lesdits scores correspondant à la projection desdites données expérimentales sur lesdites q composantes principales ;
   - sélectionner k premières composantes principales parmi les q composantes principales produites ;
   - projeter lesdites données expérimentales (Z1, ..., Zi, ..., Zn) sur les k composantes principales sélectionnées et produire des données expérimentales atténuées en bruit (Z1', ..., Zi', ..., Zn') en lien avec la pluralité de voxels (V1, ..., Vn).

   ledit système (S) étant **caractérisé en ce que** l'unité de traitement (4) est configurée pour :

   - produire des images des scores des composantes principales pour la pluralité de voxels (V1, ..., Vn) et des indicateurs informatifs ($\sigma 1^r$, ..., $\sigma q^r$) quantifiant l'information spatiale contenue dans lesdites images des scores associés auxdites composantes principales sous la forme d'un taux de diminution des valeurs d'une caractéristique déterminée desdits scores avant et après l'application d'un filtre de lissage sur lesdits scores, ladite caractéristique déterminée des scores pour une pluralité de voxels (V1, ..., Vn) appartenant à un ensemble de caractéristiques, prises seules ou en combinaison, comportant la variance, l'écart-type, la médiane et la moyenne ;
   - sélectionner les k premières composantes principales à partir desdits indicateurs informatifs produits.

10. Système (S) d'analyse d'imagerie médicale (S) comportant une unité de traitement (4) agencée pour atténuer le bruit de données expérimentales (Zi) fruits d'acquisitions multiples par un appareil d'imagerie de résonance magnétique (1) en lien avec un volume élémentaire d'intérêt, ci-après nommé « voxel » d'intérêt, parmi une pluralité de voxels, ladite unité de traitement (4) étant configurée pour :

   - collecter desdites données expérimentales (Z1, ..., Zi, ..., Zn) en lien avec la pluralité de voxels (V1, ..., Vn) ;
   - produire un ensemble ordonné, selon leurs valeurs singulières respectives, de q composantes principales ainsi que leurs scores respectifs pour la pluralité de voxels (V1, ..., Vn), lesdits scores correspondant à la projection desdites données expérimentales sur lesdites q composantes principales ;
   - sélectionner k premières composantes principales parmi les q composantes principales produites ;
   - projeter lesdites données expérimentales (Z1, ..., Zi, ..., Zn) sur les k composantes principales sélectionnées et produire des données expérimentales atténuées en bruit (Z1', ..., Zi', ..., Zn') en lien avec la pluralité de voxels (V1, ..., Vn).

   ledit système (S) étant **caractérisé en ce que** l'unité de traitement (4) est configurée pour :

   - produire des images des scores des composantes principales pour la pluralité de voxels (V1, ..., Vn) et des indicateurs informatifs ($\sigma 1^r$, ..., $\sigma q^r$) quantifiant l'information spatiale contenue dans lesdites images des scores associés auxdites composantes principales sous la forme d'un taux de diminution des valeurs d'une caractéristique déterminée desdits scores avant et après l'application d'un filtre de lissage sur lesdits scores, ladite caractéristique déterminée des scores pour une pluralité de voxels (V1, ..., Vn) appartenant à un ensemble de caractéristiques, prises seules ou en combinaison, comportant la variance, l'écart-type, la médiane et la moyenne ;

- sélectionner les k premières composantes principales à partir desdits indicateurs informatifs produits.

11. Système d'analyse d'imagerie médicale (S) selon la revendication 9 ou 10 comportant une mémoire de programmes comportant les instructions de programme d'un produit programme d'ordinateur conforme à la revendication 7.

**Patentansprüche**

1. Verfahren (100) zum Dämpfen des Rauschens in experimentellen Daten (Zi), die aus mehreren Erfassungen durch eine Magnetresonanztomographieeinrichtung (1) in Bezug auf ein Elementarvolumen von Interesse, im Folgenden als "Voxel" von Interesse bezeichnet, aus einer Vielzahl von Voxeln resultieren, wobei das Verfahren (100) durch eine Verarbeitungseinheit (4) eines medizinischen Bildanalysesystems (S) implementiert wird, das Verfahren (100) umfassend:

- einen Schritt (110) zum Sammeln der experimentellen Daten (Z1,..., Zi, ..., Zn) in Bezug auf die Vielzahl von Voxeln (V1, ..., Vn);
- einen Schritt (120) zum Erzeugen eines geordneten Satzes, gemäß ihren jeweiligen Eigenwerten, von q Hauptkomponenten sowie ihrer jeweiligen Bewertungen für die Vielzahl von Voxeln (V1, ..., Vn), wobei die Bewertungen der Projektion der experimentellen Daten auf die q Hauptkomponenten entsprechen;
- einen Schritt (130) zum Auswählen der k ersten Hauptkomponenten aus den erzeugten q Hauptkomponenten;
- einen Schritt (140) zum Projizieren der experimentellen Daten (Zi) auf die k ausgewählten Hauptkomponenten und zum Erzeugen der rauschgedämpften experimentellen Daten (Zi') in Bezug auf das Elementarvolumen von Interesse;

wobei das Verfahren **dadurch gekennzeichnet ist, dass** der Schritt (130) zum Auswählen von k Hauptkomponenten (130) des Verfahrens (100) umfasst:

- einen Unterschritt (131) zum Erzeugen von Bildern der Bewertungen der Hauptkomponenten für die Vielzahl von Voxeln (V1, ..., Vn) und von informativen Indikatoren ($\sigma 1^r$, ..., $\sigma q^r$), die die räumlichen Informationen, die in den Bildern der Bewertungen enthalten sind, die mit den Hauptkomponenten verknüpft sind, in Form einer Abnahmerate der Werte einer bestimmten Eigenschaft der Bewertungen vor und nach der Anwendung eines Glättungsfilters auf die Bewertungen quantifizieren, wobei die bestimmte Eigenschaft der Bewertungen für eine Vielzahl von Voxeln (V1, ..., Vn) zu einem Satz von Eigenschaften gehört, einzeln oder in Kombination betrachtet, der die Varianz, die Standardabweichung, den Median und den Mittelwert umfasst;
- einen Unterschritt (132) zum Bestimmen des Rangs k zum Auswählen der k ersten Hauptkomponenten aus den erzeugten informativen Indikatoren ($\sigma 1^r$, ..., $\sigma q^r$).

2. Verfahren (100) zum Dämpfen des Rauschens in experimentellen Daten (Zi), die aus mehreren Erfassungen durch eine Magnetresonanztomographieeinrichtung (1) in Bezug auf ein Elementarvolumen von Interesse, im Folgenden als "Voxel" von Interesse bezeichnet, unter einer Vielzahl von Voxeln resultieren, wobei das Verfahren (100) durch eine Verarbeitungseinheit (4) eines medizinischen Bildanalysesystems (S) implementiert wird, das Verfahren (100) umfassend:

- einen Schritt (110) zum Sammeln der experimentellen Daten (Z1,..., Zi, ..., Zn) in Bezug auf die Vielzahl von Voxeln (V1, ..., Vn);
- einen Schritt (120) zum Erzeugen eines geordneten Satzes, gemäß ihren jeweiligen Singulärwerten, von q Hauptkomponenten sowie ihrer jeweiligen Bewertungen für die Vielzahl von Voxeln (V1, ..., Vn), wobei die Bewertungen der Projektion der experimentellen Daten auf die q Hauptkomponenten entsprechen;
- einen Schritt (130) zum Auswählen der k ersten Hauptkomponenten aus den erzeugten q Hauptkomponenten;
- einen Schritt (140) zum Projizieren der experimentellen Daten (Zi) auf die k ausgewählten Hauptkomponenten und zum Erzeugen der rauschgedämpften experimentellen Daten (Zi') in Bezug auf das Elementarvolumen von Interesse;

wobei das Verfahren **dadurch gekennzeichnet ist, dass** der Schritt (130) zum Auswählen von k Hauptkomponenten (130) des Verfahrens (100) umfasst:

- einen Unterschritt (131) zum Erzeugen von Bildern der Bewertungen der Hauptkomponenten für die Vielzahl von Voxeln (V1, ..., Vn) und von informativen Indikatoren ($\sigma 1r$, ..., $\sigma qr$), die die räumlichen Informationen, die in den Bildern der Bewertungen enthalten sind, die mit den Hauptkomponenten verknüpft sind, in Form einer Abnahmerate der Werte einer bestimmten Eigenschaft der Bewertungen vor und nach der Anwendung eines Glättungsfilters auf die Bewertungen quantifizieren, wobei die bestimmte Eigenschaft der Bewertungen für eine Vielzahl von Voxeln (V1, ..., Vn) zu einem Satz von Eigenschaften gehört, einzeln oder in Kombination betrachtet, der die Varianz, die Standardabweichung, den Median und den Mittelwert umfasst;

- einen Unterschritt (132) zum Bestimmen des Rangs k zum Auswählen der k ersten Hauptkomponenten aus den erzeugten informativen Indikatoren ($\sigma$1r, ..., $\sigma$qr).

3. Verfahren (100) nach Anspruch 1 oder 2, wobei der Unterschritt (132) zum Bestimmen des Rangs k aus der Berechnung der mathematischen Differenz zwischen den Werten der informativen Indikatoren ($\sigma1^r$, ..., $\sigma q^r$) besteht, wenn diese entsprechend den erzeugten q Hauptkomponenten geordnet sind, wobei der Rang k derjenige der Hauptkomponente ist, für die die mathematische Differenz kleiner als oder gleich einem bestimmten Schwellenwert wird.

4. Verfahren (100) nach Anspruch 1 oder 2, wobei der Unterschritt (132) zum Bestimmen des Rangs k aus der Erkennung eines Plateaus ($\sigma p^r$) besteht, beschrieben durch die Werte der informativen Indikatoren ($\sigma1^r$, ..., $\sigma q^r$), wenn diese entsprechend den erzeugten q Hauptkomponenten geordnet sind, ausgehend von dem informativen Indikator ($\sigma q^r$) der Hauptkomponente des Rangs q bis zu dem informativen Indikator ($\sigma1^r$) der Hauptkomponente mit Rang 1, wobei Rang k derjenige der ersten Hauptkomponente ist, deren Wert des informativen Indikators ($\sigma k^r$) von diesem Plateau ($\sigma p^r$) abweicht.

5. Verfahren (100) nach einem der vorstehenden Ansprüche, umfassend einen Unterschritt (133) zum Anwenden einer Filteroperation der ausgewählten k Hauptkomponenten, deren Filterintensität für jede der k ausgewählten Hauptkomponenten spezifisch und proportional zu dem informativen Indikator ($\sigma1^r$, ..., $\sigma11^r$) dieses letzteren ist.

6. Verfahren (100) nach dem vorstehenden Anspruch, wobei die Filteroperation (133) aus der alleinigen oder kombinierten Verwendung eines Gauß-Filters, eines Mittelwertfilters, eines Median-Filters, eines anistropen Diffusionsfilters besteht.

7. Computerprogrammprodukt, umfassend eine oder mehrere Programmanweisungen, die durch die Verarbeitungseinheit (4) eines medizinischen Bildanalysesystems (S) interpretierbar sind, wobei die Programmanweisungen in einen nichtflüchtigen Speicher des medizinischen Bildanalysesystems (S) ladbar sind, **dadurch gekennzeichnet, dass** die Ausführung der Anweisungen durch die Verarbeitungseinheit (4) die Implementierung eines Verfahrens (100) nach einem der vorstehenden Ansprüche bewirkt.

8. Computerlesbares Speichermedium, umfassend die Anweisungen eines Computerprogrammprodukts nach dem vorstehenden Anspruch.

9. Medizinisches Bildanalysesystem (S), umfassend eine Verarbeitungseinheit (4), die zum Dämpfen des Rauschens in experimentellen Daten (Zi) eingerichtet ist, die aus mehreren Erfassungen durch eine Magnetresonanztomographieeinrichtung (1) in Bezug auf ein Elementarvolumen von Interesse, im Folgenden als "Voxel" von Interesse bezeichnet, aus einer Vielzahl von Voxeln resultieren, wobei die Verarbeitungseinheit (4) konfiguriert ist zum:

- Sammeln der experimentellen Daten (Z1,..., Zi, ..., Zn) in Bezug auf die Vielzahl von Voxeln (V1, ..., Vn);
- Erzeugen eines geordneten Satzes, gemäß ihren jeweiligen Eigenwerten, von q Hauptkomponenten sowie ihrer jeweiligen Bewertungen für die Vielzahl von Voxeln (V1, ..., Vn), wobei die Bewertungen der Projektion der experimentellen Daten auf die q Hauptkomponenten entsprechen;
- Auswählen der k ersten Hauptkomponenten aus den erzeugten q Hauptkomponenten;

　Projizieren der experimentellen Daten (Z1, ..., Zi, ..., Zn) auf die k ausgewählten Hauptkomponenten und Erzeugen der rauschgedämpften experimentellen Daten (Z1', ..., Zi', ..., Zn') in Bezug auf die Vielzahl von Voxeln (V1, ..., Vn),
　wobei das System (S) **dadurch gekennzeichnet ist, dass** die Verarbeitungseinheit (4) konfiguriert ist zum:

　　- Erzeugen von Bildern der Bewertungen der Hauptkomponenten für die Vielzahl von Voxeln (V1, ..., Vn) und der informativen Indikatoren ($\sigma1^r$, ..., $\sigma q^r$), die die räumlichen Informationen, die in den Bildern der Bewertungen enthalten sind, die mit den Hauptkomponenten verknüpft sind, in Form einer Abnahmerate der Werte einer bestimmten Eigenschaft der Bewertungen vor und nach der Anwendung eines Glättungsfilters auf die Bewertungen quantifizieren, wobei die bestimmte Eigenschaft der Bewertungen für eine Vielzahl von Voxeln (V1, ..., Vn) zu einem Satz von Eigenschaften gehört, einzeln oder in Kombination betrachtet, der die Varianz, die Standardabweichung, den Median und den Mittelwert umfasst;

- Auswählen der k ersten Hauptkomponenten aus den erzeugten informativen Indikatoren.

10. Medizinisches Bildanalysesystem (S), umfassend eine Verarbeitungseinheit (4), die zum Dämpfen des Rauschens in experimentellen Daten (Zi) eingerichtet ist, die aus mehreren Erfassungen durch eine Magnetresonanztomographieeinrichtung (1) in Bezug auf ein Elementarvolumen von Interesse, im Folgenden als "Voxel" von Interesse bezeichnet, aus einer Vielzahl von Voxeln resultieren, wobei die Verarbeitungseinheit (4) konfiguriert ist zum:

- Sammeln der experimentellen Daten (Z1,..., Zi, ..., Zn) in Bezug auf die Vielzahl von Voxeln (V1, ..., Vn);
- Erzeugen eines geordneten Satzes, gemäß ihren jeweiligen Singulärwerten, von q Hauptkomponenten sowie ihrer jeweiligen Bewertungen für die Vielzahl von Voxeln (V1, ..., Vn), wobei die Bewertungen der Projektion der experimentellen Daten auf die q Hauptkomponenten entsprechen;
- Auswählen der k ersten Hauptkomponenten aus den erzeugten q Hauptkomponenten; ·

Projizieren der experimentellen Daten (Z1, ..., Zi, ..., Zn) auf die k ausgewählten Hauptkomponenten und Erzeugen der rauschgedämpften experimentellen Daten (Z1', ..., Zi', ..., Zn') in Bezug auf die Vielzahl von Voxeln (V1, ..., Vn),
wobei das System (S) **dadurch gekennzeichnet ist, dass** die Verarbeitungseinheit (4) konfiguriert ist zum:

- Erzeugen von Bildern der Bewertungen der Hauptkomponenten für die Vielzahl von Voxeln (V1, ..., Vn) und der informativen Indikatoren ($\sigma 1^r$, ..., $\sigma q^r$), die die räumlichen Informationen, die in den Bildern der Bewertungen enthalten sind, die mit den Hauptkomponenten verknüpft sind, in Form einer Abnahmerate der Werte einer bestimmten Eigenschaft der Bewertungen vor und nach der Anwendung eines Glättungsfilters auf die Bewertungen quantifizieren, wobei die bestimmte Eigenschaft der Bewertungen für eine Vielzahl von Voxeln (V1, ..., Vn) zu einem Satz von Eigenschaften gehört, einzeln oder in Kombination betrachtet, der die Varianz, die Standardabweichung, den Median und den Mittelwert umfasst;
- Auswählen der k ersten Hauptkomponenten aus den erzeugten informativen Indikatoren.

11. Medizinisches Bildanalysesystem (S) nach Anspruch 9 oder 10, umfassend einen Programmspeicher, umfassend die Programmanweisungen eines Computerprogrammprodukts nach Anspruch 7.

**Claims**

1. Method (100) for attenuating the noise in experimental data (Zi) resulting from multiple acquisitions by a magnetic resonance imaging device (1) in relation to an elementary volume of interest, hereinafter referred to as a "voxel" of interest, among a plurality of voxels, said method (100) being implemented by a processing unit (4) of a medical imaging analysis system (S), said method (100) comprising:

- a step (110) of collecting said experimental data (Z1,... , Zi, ... , Zn) in relation to the plurality of voxels (V1, ... , Vn);
- a step (120) of producing an ordered set of q main components, according to their respective eigenvalues, and their respective scores for the plurality of voxels (V1, ..., Vn), said scores corresponding to the projection of said experimental data onto said q main components;
- a step (130) of selecting the k first main components from among the q main components produced;
- a step (140) of projecting said experimental data (Zi) onto the k selected main components and producing noise-attenuated experimental data (Zi') in relation to the elementary volume of interest;

said method being **characterized in that** the step (130) of selecting k main components (130) of said method (100) comprises:

- a sub-step (131) of producing main component score images for the plurality of voxels (V1, ... , Vn) and informative indicators ($\sigma 1^r$, ... , $\sigma q^r$) quantifying the spatial information contained in said images of the scores associated with said main components in the form of a rate of decrease of the values of a determined characteristic of said scores before and after the application of a smoothing filter to said scores, said determined characteristic of the scores for a plurality of voxels (V1, ... , Vn) belonging to a set of characteristics, taken alone or in combination, including variance, standard deviation, median and mean;

- a sub-step (132) of determining the rank k in order to select the k first main components from said informative indicators ($\sigma 1^r$, ... , $\sigma q^r$) produced.

2. Method (100) for attenuating the noise in experimental data (Zi) resulting from multiple acquisitions by a magnetic resonance imaging device (1) in relation to an elementary volume of interest, hereinafter referred to as a "voxel" of interest, among a plurality of voxels, said method (100) being implemented by a processing unit (4) of a medical imaging analysis system (S), said method (100) comprising:

- a step (110) of collecting said experimental data (Z1, ... , Zi, ... , Zn) in relation to the plurality of voxels (V1, ... , Vn);
- a step (120) of producing an ordered set of q main components, according to their respective singular values, and their respective scores for the plurality of voxels (V1, ... , Vn), said scores corresponding to the projection of said experimental data onto said q main components;
- a step (130) of selecting the k first main components from among the q main components produced;
- a step (140) of projecting said experimental data (Zi) onto the k selected main components and producing noise-attenuated experimental data (Zi') in relation to the elementary volume of interest;

said method being **characterized in that** the step (130) of selecting k main components (130) of said method (100) comprises:

- a sub-step (131) of producing main component score images for the plurality of voxels (V1, ... , Vn) and informative indicators ($\sigma 1r$, ..., $\sigma qr$) quantifying the spatial information contained in said images of the scores associated with said main components in the form of a rate of decrease of the values of a determined characteristic of said scores before and after the application of a smoothing filter to said scores, said determined characteristic of the scores for a plurality of voxels (V1, ... , Vn) belonging to a set of characteristics, taken alone or in combination, including variance, standard deviation, median and mean;
- a sub-step (132) of determining the rank k in order to select the k first main components from said informative indicators ($\sigma 1r$, ... , $\sigma qr$) produced.

3. Method (100) according to claim 1 or 2, for which the sub-step (132) of determining the rank k consists in calculating the mathematical difference between values of said informative indicators ($\sigma 1^r$, ... , $\sigma q^r$) when the indicators are ordered according to the q main components produced, with rank k being that of the main component for which said mathematical difference becomes less than or equal to a given threshold.

4. Method (100) according to claim 1 or 2, for which the sub-step (132) of determining the rank k consists in detecting a plateau ($\sigma p^r$) described by the values of the informative indicators ($\sigma 1^r$, ... , $\sigma q^r$) when the indicators are ordered according to the q main components produced, from the informative indicator ($\sigma q^r$) of the main component of rank q to the informative indicator ($\sigma 1^r$) of the main component of rank 1, with rank k being that of the first main component of which the informative indicator value ($\sigma k^r$) deviates from said plateau ($\sigma p^r$).

5. Method (100) according to any one of the preceding claims, comprising a sub-step (133) of applying a filtering operation to the k selected main components, the filtering intensity of which is specific to each of the k selected main components and proportional to the informative indicator ($\sigma 1^r$, ... , $\sigma 11^r$) of said component.

6. Method (100) according to the preceding claim, for which the filtering operation (133) consists in the single or combined use of a Gaussian filter, a mean filter, a median filter or an anisotropic diffusion filter.

7. Computer program product comprising one or more program instructions which can be interpreted by the processing unit (4) of a medical imaging analysis system (S), said program instructions being loadable into a non-volatile memory of said medical imaging analysis system (S), **characterized in that** the execution of said instructions by said processing unit (4) causes the implementation of a method (100) according to any one of the preceding claims.

8. Computer-readable storage medium containing instructions for a computer program product according to the preceding claim.

9. Medical imaging analysis system (S) comprising a processing unit (4) arranged for attenuating the noise in experimental data (Zi) resulting from multiple acquisitions by a magnetic resonance imaging device (1) in relation to an elementary volume of interest, hereinafter referred to as a "voxel" of interest, among a plurality of voxels, said processing unit (4) being configured to:

- collect said experimental data (Z1, ... , Zi, ... , Zn) in relation to the plurality of voxels (V1, ... , Vn);

- produce an ordered set of q main components, according to their respective eigenvalues, and their respective scores for the plurality of voxels (V1, ... , Vn), said scores corresponding to the projection of said experimental data onto said q main components;
- select k first main components from among the q main components produced;
- project said experimental data (Z1, ... , Zi, ... , Zn) onto the k selected main components and produce noise-attenuated experimental data (Z1', ... , Zi', ... , Zn') in relation to the plurality of voxels (V1, ... , Vn).

said system (S) being **characterized in that** the processing unit (4) is configured to:

- produce main component score images for the plurality of voxels (V1, ... , Vn) and informative indicators ($\sigma 1^r$, ... , $\sigma q^r$) quantifying the spatial information contained in said images of the scores associated with said main components in the form of a rate of decrease of the values of a determined characteristic of said scores before and after the application of a smoothing filter to said scores, said determined characteristic of the scores for a plurality of voxels (V1, ... , Vn) belonging to a set of characteristics, taken alone or in combination, including variance, standard deviation, median and mean;
- select the k first main components from said informative indicators produced.

10. Medical imaging analysis system (S) comprising a processing unit (4) arranged for attenuating the noise in experimental data (Zi) resulting from multiple acquisitions by a magnetic resonance imaging device (1) in relation to an elementary volume of interest, hereinafter referred to as a "voxel" of interest, among a plurality of voxels, said processing unit (4) being configured to:

- collect said experimental data (Z1, ... , Zi, ..., Zn) in relation to the plurality of voxels (V1, ..., Vn);
- produce an ordered set of q main components, according to their respective singular values, and their respective scores for the plurality of voxels (V1, ... , Vn), said scores corresponding to the projection of said experimental data onto said q main components;
- select k first main components from among the q main components produced;
- project said experimental data (Z1, ... , Zi, ... , Zn) onto the k selected main components and produce noise-attenuated experimental data (Z1', ... , Zi', ... , Zn') in relation to the plurality of voxels (V1, ... , Vn).

said system (S) being **characterized in that** the processing unit (4) is configured to:

- produce main component score images for the plurality of voxels (V1, ... , Vn) and informative indicators ($\sigma 1^r$, ... , $\sigma q^r$) quantifying the spatial information contained in said images of the scores associated with said main components in the form of a rate of decrease of the values of a determined characteristic of said scores before and after the application of a smoothing filter to said scores, said determined characteristic of the scores for a plurality of voxels (V1, ... , Vn) belonging to a set of characteristics, taken alone or in combination, including variance, standard deviation, median and mean;
- select the k first main components from said informative indicators produced.

11. Medical imaging analysis system (S) according to claim 9 or 10, comprising a program memory containing the program instructions of a computer program product according to claim 7.

FIG.1

FIG.2

$$Zi\ (\Delta\omega) = \frac{M(\Delta\omega)}{M0}$$

FIG.3

FIG.4

$$\Phi = \{\varphi 1, \ldots, \varphi j, \ldots, \varphi q\}$$
$$\Lambda = \{\lambda 1, \ldots, \lambda j, \ldots, \lambda q\}$$

FIG. 5

FIG. 6

FIG. 7

$$\Phi = \{\varphi 1, \ldots, \varphi j, \ldots, \varphi q\}$$
$$\Phi' = \{\varphi 1, \ldots, \varphi k\}\, k \leq q$$
$$U = \{u1, \ldots, uq\}$$
$$U' = \{u1, \ldots, uk\}\, k \leq q$$
$$\Sigma^r = \{\sigma 1^r, \ldots, \sigma q^r\}$$

$$\sigma 9^r = \frac{\sigma 9^a - \sigma 9^b}{\sigma 9^a}$$

$$\sigma 3^r = \frac{\sigma 3^a - \sigma 3^b}{\sigma 3^a}$$

$$\sigma 29^r = \frac{\sigma 29^a - \sigma 29^b}{\sigma 29^a}$$

FIG. 8

FIG. 9

FIG. 10

FIG. 11

FIG. 12

FIG. 13

FIG. 14

$$wj^r = \frac{\sigma j^r - \sigma 1^r}{\sigma p^r - \sigma 1^r}$$

FIG. 15

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **CÁRDENAS-BLANCO, ARTURO** ; **CRISTIAN TEJOS** ; **PABLO IRARRAZAVAL** ; **LAN CAMERON**. Noise in Magnitude Magnetic Resonance Images.. *Concepts in Magnetic Resonance Part A*, 2008, vol. 32A (6), 409-16, https://doi.org/10.1002/cmr.a.20124
- **JOLLIFFE, I**. Principal Component Analysis. Springer-Verlag, 2002
- **BREITLING, JOHANNES** ; **ANAGHA DESHMANE** ; **STEFFEN GOERKE** ; **ANDREAS KORZOWSKI** ; **KAI HERZ** ; **MARK E. LADD** ; **KLAUS SCHEFFLER** ; **PETER BACHERT** ; **MORITZ ZAISS**. Adaptive Denoising for Chemical Exchange Saturation Transfer MR Imaging.. *NMR in Biomedicine*, March 2019, 1-14, https://doi.org/10.1002/nbm.4133
- **KAISER, HENRY F.** The Varimax Criterion for Analytic Rotation in Factor Analysis.. *Psychometrika*, 1958, vol. 23 (3), 187-200, https://doi.org/10.1007/BF02289233

- **WEIHUA LI** ; **S. JOE QIN**. Selection of the Number of Principal Components: The Variance of the Reconstruction Error Criterion with a Comparison to Other Methods.. *Industrial and Engineering Chemistry Research*, 1999, vol. 38 (11), 4389-4401
- **VERAART, JELLE** ; **DMITRY S. NOVIKOV** ; **DAAN CHRISTIAENS** ; **BENJAMIN ADES-ARON** ; **JAN SIJBERS** ; **ELS FIEREMANS**. Denoising of Diffusion MRI Using Random Matrix Theory.. *NeuroImage*, 2016, vol. 142, 394-406, https://doi.org/10.1016/j.neuroimage.2016.08.016
- **GAVISH, MATAN** ; **DAVID L.** Optimal Shrinkage of Singular Values.. *IEEE Transactions on Information Theory*, 2017, vol. 63 (4), 2137-52, https://doi.org/10.1109/TIT.2017.2653801